(19) Europäisches Patentamt
European Patent Office
Office européen des brevets

(11) Veröffentlichungsnummer: **0 086 173**
**B1**

(12) # EUROPÄISCHE PATENTSCHRIFT

(45) Veröffentlichungstag der Patentschrift: **03.09.86**

(21) Anmeldenummer: **83810007.1**

(22) Anmeldetag: **10.01.83**

(51) Int. Cl.⁴: **C 07 D 233/60,** C 07 D 249/08, A 01 N 43/653, A 01 N 43/50 // C07D303/22

(54) **Fungizid wirkende und den Pflanzenwuchs regulierende Triazolcarbinolderivate.**

(30) Priorität: **15.01.82 CH 240/82**

(43) Veröffentlichungstag der Anmeldung: **17.08.83 Patentblatt 83/33**

(45) Bekanntmachung des Hinweises auf die Patenterteilung: **03.09.86 Patentblatt 86/36**

(84) Benannte Vertragsstaaten: **BE CH DE FR GB IT LI NL**

(56) Entgegenhaltungen:
**EP-A-0 052 424**
**EP-A-0 061 835**
**US-A-4 036 970**
**US-A-4 036 973**
**US-A-4 036 974**

(73) Patentinhaber: **CIBA- GEIGY AG, Klybeckstrasse 141, CH- 4002 Basel (CH)**

(72) Erfinder: **Meyer, Alfred, Dr., Bristenweg 6, CH- 4054 Basel (CH)**
Erfinder: **Rempfler, Hermann, Dr., Brücklismattstrasse 16, CH- 4107 Ettingen (CH)**
Erfinder: **Nyfeler, Robert, Dr., Bärenfelserstrasse 8, CH- 4057 Basel (CH)**
Erfinder: **Sturm, Elmar, Dr., Klusstrasse 66, CH- 4147 Aesch (CH)**
Erfinder: **Müller, Urs, Dr., Schluchtstrasse 15, CH- 4142 Münchenstein (CH)**

EP 0 086 173 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft neue, substituierte 2-Hydroxy-3-triazolyl-propan-derivate, deren Säureadditionssalze, quaternäre Azoliumsalze und Metallkomplexe. Die Erfindung betrifft ferner die Herstellung dieser Substanzen sowie fungizide und pflanzenwuchsregulierende Mittel, die als Wirkstoff mindestens eine dieser Verbindungen enthalten. Die Erfindung betrifft auch die Herstellung der genannten Mittel und die Verwendung der Wirkstoffe oder der Mittel zur Regulierung des Pflanzenwachstums und zur Bekämpfung von schädlichen Mikroorganismen.

Es werden hierin Verbindungen der allgemeinen Formel I

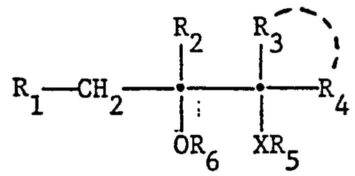

(I)

umfasst, worin

$R_1$ einen über Stickstoff gebundenen Di- oder Triazolrest,

$R_2$ $C_1$-$C_{10}$ Alkyl, $C_3$-$C_{10}$ Cycloalkyl, einen unsubstituiertenoder im Kern durch Halogen, Cyan, $C_1$-$C_3$ Alkyl, $C_1$-$C_3$ Haloalkyl, $C_1$-$C_3$ Alkoxy, $C_1$-$C_3$ Haloalkoxy, $C_1$-$C_3$ Alkylthio, $C_1$-$C_3$ Haloalkylthio, Nitro und/oder Rhodano substituierten Phenyl-, Naphthyl-, Biphenyl-, Phenoxyphenyl- oder Benzylrest

$R_3$ und $R_4$ unabhängig voneinander je Wasserstoff, $C_1$-$C_4$ Alkyl oder zusammen eine 2-10 gliedrige Alkylenbrücke,

$R_5$ einen unsubstituierten oder ein- oder mehrfach substituierten Rest, ausgewählt aus der Reihe $C_1$-$C_8$ Alkyl, $C_3$-$C_8$ Cycloalkyl, $C_3$-$C_6$ Alkenyl, $C_3$-$C_6$-Alkinyl, Phenyl, Naphthyl, Biphenyl, Benzylphenyl, Benzyloxyphenyl, Phenoxyphenyl und Benzyl, wobei die Substituenten aus der Reihe Halogen, Cyano, $C_1$-$C_3$ Alkyl, $C_1$-$C_5$ Alkoxy, $C_1$-$C_5$ Haloalkoxy, $C_1$-$C_3$ Alkylthio, $C_1$-$C_3$ Haloalkyl, $C_1$-$C_3$ Haloalkylthio, Nitro und/oder Rhodano ausgewählt sind,

$R_6$ $C_1$-$C_{10}$ Alkyl, unsubstituiert oder substituiert durch Halogen, Cyan, $C_1$-$C_4$ Alkoxy, $C_1$-$C_4$ Alkylcarbonyl, $C_1$-$C_4$ Alkoxycarbonyl, $C_1$-$C_4$ Alkylthio oder $C_1$-$C_4$ Alkylthiocarbonyl;

$C_3$-$C_4$ Alkenyl, $C_3$-$C_4$ Alkinyl, $C_3$-$C_8$ Cycloalkyl;

einen im Kern durch Halogen, Cyan, $C_1$-$C_3$ Alkyl, $C_1$-$C_3$ Haloalkyl, $C_1$-$C_3$ Alkoxy, $C_1$-$C_3$ Haloalkoxy, $C_1$-$C_3$ Alkylthio, $C_1$-$C_3$ Haloalkylthio, Nitro und/oder Rhodano substituierten Benzylrest oder einen Rest -Si($C_1$-$C_4$ Alkyl$)_3$, $-CONR_7R_8$, $-COR_9$, $-SO_2NR_7R_8$ oder $-SO_2R_{10}$,

worin

$R_7$ und $R_8$ je einzelnen Wasserstoff oder $C_1$-$C_6$ Alkyl oder zusammen mit dem Stickstoffatom, an das sie gebunden sind einen gesättigten 5-6 gliedrigen Heterocyclus, der auch Sauerstoff, Schwefel die Imino oder eine $C_1$-$C_4$ Alkyliminogruppe enthalten kann,

$R_9$ $C_1$-$C_{10}$ Alkyl unsubstituiert oder substituiert durch Halogen, Cyan, $C_1$-$C_4$ Alkoxy, $C_1$-$C_4$ Alkylcarbonyl, $C_1$-$C_4$ Alkoxycarbonyl, Phenyl oder $C_3$-$C_8$ Cycloalkyl, oder einen unsubstituierten oder durch Halogen, Cyan, $C_1$-$C_3$ Alkyl, $C_1$-$C_3$ Haloalkyl, $C_1$-$C_3$ Alkoxy, $C_1$-$C_3$ Haloalkoxy, $C_1$-$C_3$ Alkylthio, $C_1$-$C_3$ Haloalkylthio, Nitro und/oder Rhodano substituierten Phenylrest,

$R_{10}$ $C_1$-$C_4$ Alkyl oder Phenyl, unsubstituiert oder substituiert durch Halogen, Cyan, $C_1$-$C_3$ Alkyl, C -$C_3$ Alkoxy, $C_1$-$C_3$ Haloalkoxy, $C_1$-$C_3$ Alkylthio, $C_1$-$C_3$ Haloalkylthio, Nitro und/oder Rhodano und

X Sauerstoff oder Schwefel bedeutet. unter Einschluss der Säureadditionssalze, quaternären Azoliumsalze und Metallkomplexe, mit Ausnahme von 1[1H-1,2,4-Triazol]-2-methoxy-2-tert.butyl-3-(4-fluorphenoxy)-propan.

Unter Triazolen werden heterocyclische Fünfringe mit Stickstoffen als Heteroatome und mit aromatischem Charakter verstanden. Die Vertreter sind 1H-1,2,4-Triazol und 4H-1,2,4-Triazol. Unter dem Begriff Alkyl selbst oder als Bestandteil eines anderen Substituenten sind je nach Zahl der angegebenen Kohlenstoffatome beispeilsweise folgende Gruppen zu verstehen: Methyl, Ethyl, Propyl, Butyl, Pentyl, Hexyl, Heptyl, Octyl usw. sowie ihre Isomeren, wie z.B. Isopropyl, Isobutyl, tert.-Butyl, Isopentyl usw.. Haloalkyl steht für einen einfach bis perhalogenierten Alkylsubstituenten, wie z.B. $CHCl_2$, $CHF_2$, $CH_2Cl$, $CCl_3$, $CH_2F$, $CH_2CH_2Cl$, $CHBr_2$ usw.. Unter Halogen soll hier und im folgenden Fluor, Chlor, Brom oder Jod, vorzugsweise Fluor, Chlor oder Brom, verstanden werden. Naphthyl steht für α- oder β-Naphthyl, vorzugsweise α-Naphthyl. Der Ausdruck Alkylen steht für eine unverzweigte oder verzweigte Alkylenbrücke wie z.B. Methylen, Ethylen, Propylen usw., bevorzugt für ein Brückenglied mit 4 bis 6 Kohlenstoffatomen, Alkenyl bedeutet z.B. Propenyl-(1), Allyl, Butenyl-(2) oder Butenyl-(3), Alkinyl steht z.B. für Propargyl. Aryl bedeutet z.B. Naphthyl, insbesondere Phenyl - und Benzylrest. Cycloalkyl bedeutet je nach Zahl der Kohlenstoffatome z.B. Cyclopropyl, Cyclobutyl, Cyclopentyl, Cycloheptyl, Cyclooctyl usw.

Die vorliegende Erfindung betrifft somit die freien organischen Moleküle der Formel I, deren Säureadditionssalze, quaternären Azoliumsalze und Metallkomplexe. Die freien Moleküle sind bevorzugt.

Beispiele salzbildender Säuren sind anorganische Säuren: Halogenwasserstoffsäure wie

Fluorwasserstoffsäure, Chlorwasserstoffsäure, Bromwasserstoffsäure oder Jodwasserstoffsäure sowie Schwefelsäure, Phosphorsäure, phosphorige Säure, Salpetersäure und organische Säuren wie Essigsäure, Trifluoressigsäure, Trichloressigsäure, Propionsäure, Clycolsäure, Thiocyansäure, Milchsäure, Bernsteinsäure, Zitronensäure, Benzoesäure, Zimtsäure, Oxalsäure, Ameisensäure, Benzolsulfonsäure, p-Toluolsulfonsäure, Methansulfonsäure, Salicylsäure, p-Aminosalicylsäure, 2-Phenoxybenzoesäure oder 2-Acetoxyben-zoesaure.

Metallkomplexe der Formel 1 bestehen aus dem zugrundeliegenden organischen Molekül und einem anorganischen oder organischen Metallsalz, beispielsweise den Halogeniden, Nitraten, Sulfaten, Phosphaten, Acetaten, Trifluoracetaten, Trichloracetaten, Propionaten, Tartraten, Sulfonaten, Salicylaten, Benzoaten usw. der Elemente der dritten und vierten Hauptgruppe wie Aluminium, Zinn oder Blei sowie der ersten bis achten Nebengruppe wie Chrom, Mangan, Eisen, Kobalt, Nickel, Kupfer, Zink, Silber, Quecksilber usw. Bevorzugt sind die Nebengruppen-Elemente der 4. Periode. Die Metalle können dabei in den verschiedenen ihnen zukommenden Wertigkeiten vorliegen. Die Metallkomplexe der Formel 1 können ein- oder mehrkernig auftreten, d.h. sie können ein oder mehrere organische Molekülanteile als Liganden enthalten. Komplexe mit den Metallem Kupfer, Zink, Mangan und Zinn sind bevorzugt.

Die Verbindungen der Formel I sind bei Raumtemperatur stabile Oele, Harze oder überwiegend Feststoffe, die sich durch sehr wertvolle mikrobizide und wuchsregulierende Eigenschaften auszeichnen. Sie lassen sich auf dem Agrarsektor oder verwandten Gebieten präventiv und kurativ zur Bekämpfung von pflanzenschädigenden Mikroorganismen und zur Regulierung des Pflanzenwuchses einsetzen, dabei sind die Triazolylmethylderivate im Umfang der Formel 1 bevorzugt. Die erfindungsgemässen Wirkstoffe der Formel 1 zeichnen sich durch eine sehr gute Verträglichkeit bei Kulturpflanzen aus.

In US-A-4 036 970 sind 1-Imidazol-2,3-propan diolund dithioläther beschrieben, welche durch Phenyl- und Benzylreste veräthert sind. Die US-A-4 036 973 und US-A-4 036 974 beschreiben 1-Imidazol-2,3-propan diol- und dithioläther, welche durch Alkyl und Cyclohexyl(alkyl)reste veräthert sind. Diese Verbindungen wirken gegen Pilze, Bakterien und Protozoen. In der EP-A 52 424 sind l-Imidazol- und 1-Triazolbutan, -buten - und -butinverbindungen mit fungizider Wirkung beschrieben. Die EP-A-61 835 beschreibt 1-Imidazol- und 1-Tria-zol-propan-2-ol, -3-ol oder -thiole, die veräthert sein können, mit phytofungiziden Eigenschaften. Die Verbindung 1-(1H-1,2,4-Triazol)-2-methoxy-2-tert.butyl-3-(4-fluorphenoxy)-propan ist dort ausdrücklich genannt, während die übrigen im einzelnen aufgeführten Verbindungen 1-(Di und Triazol)-propan-2-ole sind.

Auf Grund ihrer Wirkung als Fungizide oder ihren den Pflanzenwuchs regulierenden Eigenschaften sind folgende Gruppen von Wirksubstanzen der Formel Ia bevorzugt:

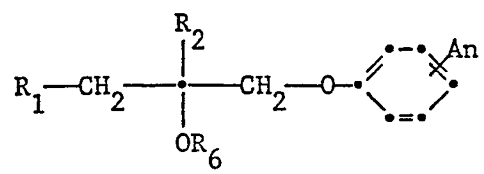

(Ia)

worin $R_1$, $R_2$ und $R_6$ die unter Formel I gegebene Bedeutung haben und A Halogen, Cyan, $C_1$-$C_3$ Alkyl, $C_1$-$C_3$ Haloalkyl, $C_1$-$C_3$ Alkoxy, $C_1$-$C_3$ Haloalkoxy, $C_1$-$C_3$ Alkylthio, $C_1$-$C_3$ Haloalkylthio, Nitro und/oder Rhodano, während

n Null oder eine Zahl von 1 bis 3 bedeutet;

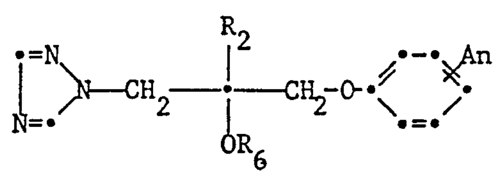

(Ib)

worin A, n, $R_2$ und $R_6$ die oben gegebene Bedeutung haben;

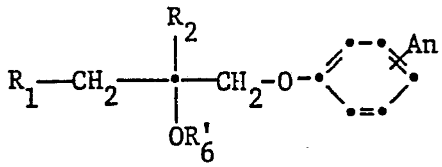

(Ic)

worin A, n, $R_1$ und $R_2$ die oben gegebene Bedeutung haben, während $R'_6$ $C_1$-$C_{10}$ Alkyl unsubstituiert oder substituiert durch Halogen, Cyan, $C_1$-$C_4$ Alkoxy, $C_1$-$C_4$ Alkoxycarbonyl, $C_1$-$C_4$ Alkylthio oder $C_1$-$C_4$ Alkylthiocarbonyl oder einen im Kern durch Halogen, Cyan, $C_1$-$C_3$ Alkyl, $C_1$-$C_3$ Haloalkyl, $C_1$-$C_3$ Alkoxy, $C_1$-$C_3$ Haloalkoxy, $C_1$-$C_3$ Alkylthio, $C_1$-$C_3$ Haloalkylthio, Nitro und/oder Rhodano substituierten Benzyl bedeutet;

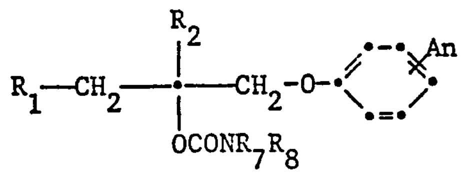

(Id)

worin A, n, $R_1$, $R_2$, $R_7$ und $R_8$, die oben gegebene Bedeutung haben;

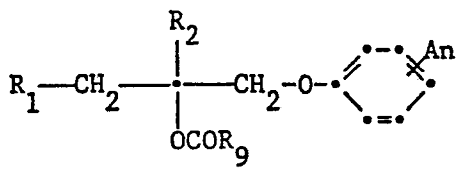

(Ie)

worin A, n, $R_1$, $R_2$, und $R_9$ die oben gegebne Bedeutung haben;

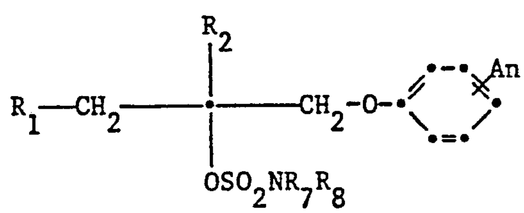

(If)

worin A, n, $R_1$, $R_2$, $R_7$ und $R_8$ die oben gegebene Bedeutung haben;

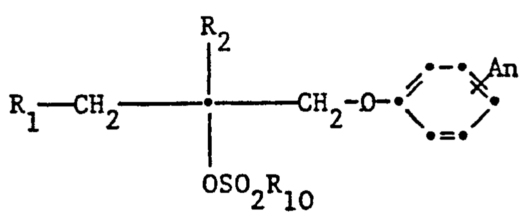

(Ig)

worin A, n, $R_2$ und $R_{10}$ die oben gegebene Bedeutung haben;

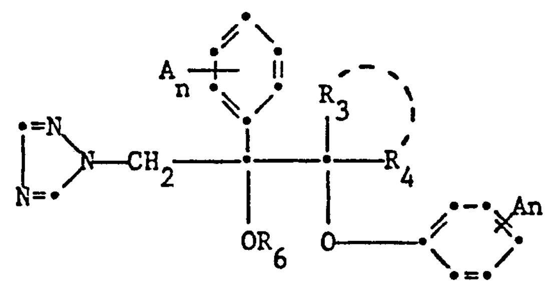

(Ih)

worin A, n, $R_3$, $R_4$ und $R_6$ die oben gegeben Bedeutung haben;

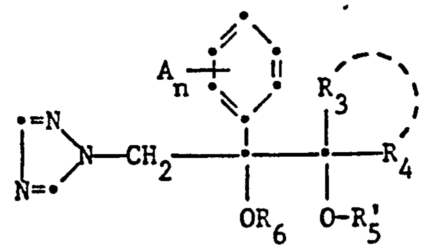

(Ii)

worin A, n. $R_3$, $R_4$ und $R_6$ die oben gegebene Bedeutung haben, während $R_5$' einen unsubstituierten oder durch Halogen, Cyan, $C_1$ -$C_3$ Alkyl, $C_1$-$C_3$ Haloalkyl, $C_1$-$C_3$ Alkoxy, $C_1$-$C_3$ Haloalkoxy, $C_1$-$C_3$ Alkylthio, $C_1$-$C_3$ Haloalkylthio, Nitro und Rhodano substituierten $C_1$ -$C_8$ Alkyl-, $C_3$-$C_8$ Cycloalkyl-, $C_3$-$C_6$ Alkenyl-, $C_3$-$C_6$ Alkinyl- oderBenzylrest bedeutet;

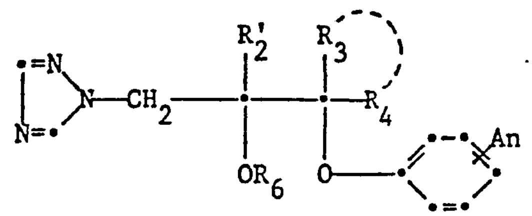

(Ij)

worin A, n, $R_3$, $R_4$ und $R_6$ die oben gegebene Bedeutung haben, während $R_2$' einen $C_1$-$C_{10}$ Alkyl- oder $C_3$-$C_{10}$ Cycloalkylrest bedeutet;

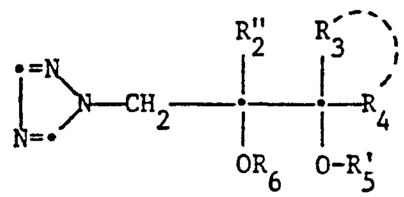

(Ik)

worin $R_3$, $R_4$, $R_5$' und $R_6$ die oben gegebene Bedeutung haben, während R''$_2$ einen $C_1$-$C_{10}$ Alkyl- oder $C_3$-$C_{10}$ Cycloalkylrest bedeutet;

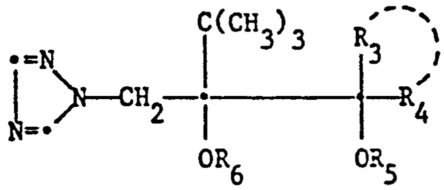

(I 1)

worin $R_3$, $R_4$, $R_5$ und $R_6$ die oben gegebene Bedeutung haben;

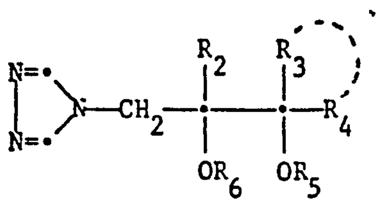

(Im)

und

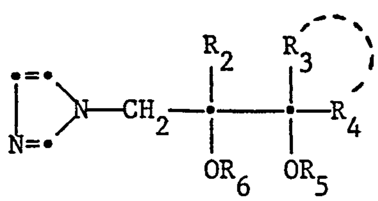

(In)

worin $R_2$, $R_3$, $R_4$, $R_5$ und $R_6$ die oben gegebene Bedeutungenhaben.
Speziell wirksam sind die Verbindungen:
1-(4-Chlorphenoxy)-2-tert.-butyl-2-(N-Methylcarbamoyloxy)-3-(1H-1,2,4-triazol)-propan,
1-(4-Fluorphenoxy)-2-tert.-butyl-2-acetyloxy-3-(1H-1,2,4-triazolyl)-propan,
1-(4-Fluorphenoxy)-2-tert.-butyl-2-benzyloxy-3-(1H-1,2,4-triazolyl)-propan,
1-(4-Chlorphenoxy)-2-tert.-butyl-2-acetyloxy-3-(1H-1,3,4-triazolyl)-propan,
1-(4-Chlorphenoxy)-2-tert.-butyl-2-methoxy-3-(1H-1,2,4-triazolyl)-propan,
1-(4-Isopropylphenoxy)-2-tert.-butyl-2-methoxy-3-(1H-1,2,4-triazolyl)-propan,
1-(4-Bromphenoxy)-2-tert.-butyl-2-allyloxy-3-(1H-1,2,4-triazolyl)-propan,
1-(4-Chlorphenoxy)-2-(4-fluorphenyl)-2-methoxy-3-(1H-1,2,4-triazolyl)-propan,
1-(1H-1,2,4-triazolyl)-2-(2,4-dichlorphenyl)-2-methoxy-3-(4-Chlor-phenoxy)-pentan,
1-n-Butoxy-2-(2,4-dichlorphenyl)-2-benzyloxy-3-(1H-1,2,4-triazolyl)-propan.
Die Verbindungen der Formel I werden in an sich bekannter Weise durch Reaktion eines Oxirans

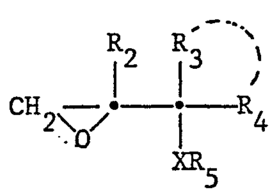

(II) ,

worin $R_2$, $R_3$, $R_4$, X und $R_5$ die unter Formel I angegebenen Bedeutungen haben, mit einem Triazol der Formel III
M - $R_1$ (III),
worin M für Wasserstoff oder bevorzugt ein Metallatom, insbesondere ein Alkalimetallatom, bedeutet und $R_1$ die unter Formel I gegebene Bedeutung hat, und Umsetzen des entstandenen 2-Hydroxy-3-triazolyl-propanderivates der Formel IV

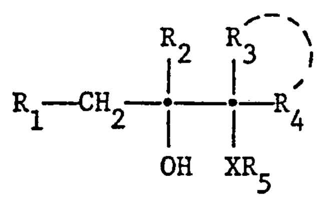

(IV) ,

worin $R_1$, $R_2$, $R_3$, $R_4$ und $R_5$ die unter Formel I gegebene Bedeutung haben mit einem reaktionsfähigen Rest der Formel V

Y - $R_6$ (V),

worin $R_6$ die obem gegebene Bedeutung hat und Y ein Halogenatom oder einen organischen oder anorganischen Säurerest bedeutet.

Diese Umsetzung wird vorteilhafterweise in einem inerten organischen Lösungs- oder Verdünnungsmittel vorgenommen und in Gegenwart von Kondensationsmitteln oder von säurebindenden Mitteln. Als solche kommen organische und anorganische Basen in Betracht, z.B. tertiäre Amine wie Trialkylamine (Trimethylamin, Triethylamin, Tripropylamin usw.), Pyridin und Pyridinbasen (4-Dimethylaminopyridin, 4-Pyrrolidyl-aminopyridin usw.), Oxide, Hydride und Hydroxide, Carbonate und Hydrogencarbonate von Alkali- und Erdalkalimetallen (CaO BaO NaOH, KOH, NaH, $Ca(OH)_2$, $KHCO_3$ $NaHCO_3$ $Ca(HCO_3)_2$, $K_2CO_3$, $Na_2CO_3$), sowie Alkaliacetate wie $CH_3COONa$ oder $CH_3COOK$. Darüberhinaus eignen sich auch Alkalialkoholate wie $C_2H_5ONa$, $C_3H_7$-nONa usw. In einigen Fällen kann es von Vorteil sein, wenn man das freie Azol III (M = Wasserstoff) zuerst, z.B. in situ mit einem Alkoholat, in das entsprechende Salz überführt und anschliessend in Gegenwart einer der genannten Basen mit dem Oxiran der Formel II umsetzt. Bei der Herstellung der 1,2,4-Triazolylderivate entstehen im allgemeinen parallel auch 1,3,4-Triazolylisomere, die sich auf übliche Weise, z.B. mit unterschiedlichen Lösungsmitteln, voneinander trennen lassen. Entstehender Halogenwasserstoff kann in manchen Fällen auch mittels Durchleiten von Inertgas, z.B. Stickstoff, aus dem Reaktionsgemisch vertrieben oder durch Zugabe von Molekularsieb absorbiert werden.

Die Reaktion (II mit III) wird bevorzugt in einem relativ polaren, jedoch reaktionsinerten organischen Lösungsmittel, durchgeführt, z.B. N,N-Dimethylformamid, N,N-Dimethylacetamid, Dimethylsulfoxid, Acetomitril, Benzonitril und anderen. Derartige Lösungsmittel können in Kombination mit anderen reaktionsinerten Lösungsmitteln, z.B. Benzol, Toluol, Xylol, Hexan, Petrolether, Chlorbenzol, Nitrobenzol u.a. durchgeführt werden. Die Reaktionstemperaturen liegen in einem Temperaturbereich von 0° bis 150°C, vorzugsweise 20° bis 100°C.

Im übrigen kann diese Reaktion analog zu bereits bekannten Umsetzungen von anderen Oxiranen mit Azolen [vgl. EP-A-0 015 756 und DE-A-29 12 288] durchgeführt werden.

Die Oxirane der Formel II sind teilweise neu, und werden nach bekannten Methoden hergestellt, siehe z.B. die EP-A-40 345.

Bei der Herstellung aller hierin genannten Ausgangs-, Zwischen- und Endprodukte können grundsätzlich, sofern nicht ausdrücklich im einzelnen spezifiziert, ein oder mehrere reaktionsinerte Lösungs- oder Verdünnungsmittel anwesend sein. In Frage kommen beispielsweise aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid; Dimethylsulfoxid; Ketone wie Aceton, Diethylketon, Methylethylketon und Gemische solcher Lösungsmittel untereinander. In manchen Fällen kann es auch von Vorteil sein, wenn die Reaktion oder Teilschritte einer Reaktion unter Schutzgasatmosphäre und/oder absoluten Lösungsmitteln durchgeführt werden. Als Schutzgase eignen sich inerte Gase wie Stickstoff, Helium, Argon oder in gewissen Fällen auch Kohlendioxid.

Das beschriebene Herstellungsverfahren ist, einschliesslich aller Teilschnitte, ein wichtiger Bestandteil der vorliegenden Erfindung.

Es wurde nun überraschend gefunden, dass die neuen Wirkstoffe der Formel I bzw. Mittel, die diese Wirkstoffe enthalten, sich vor allem dadurch auszeichnen, dass sie gezielt in den Metabolismus der Pflanzen eingreifen. Dieser gezielte Eingriff in die physiologischen Vorgänge der Pflanzenentwicklung macht die Wirkstoffe der Formel I für verschiedene Zwecke verwendbar, insbesondere für solche, die mit der Ertragssteigerung bei Nutzpflanzen, der Ernteerleichterung und der Arbeitseinsparung bei Massnahmen an Pflanzenkulturen im Zusammenhang stehen.

Für die Wirkungsweise von Pflanzenwachstumsregulatoren gilt nach der bisherigen Erfahrung, dass ein Wirkstoff eine oder auch mehrere verschiedenartige Wirkungen auf Pflanzen ausüben kann. Die Wirkungen der Stoffe hängen im wesentlichen ab von dem Zeitpunkt der Anwendung, bezogen auf das Entwicklungsstadium des Samens oder der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in gewünschter Weise positiv beeinflussen.

Pflanzenwuchsregulierende Stoffe können zum Beispiel zur Hemmung des vegetativen Pflanzenwachstums

eingesetzt werden. Eine derartige Wuchshemmung ist unter anderem bei Gräsern von wirtschaftlichem Interesse, denn dadurch kann z.B. die Häufigkeit der Grasschnitte in Ziergärten, Park- und Sportanlagen oder an Strassenrändern reduziert werden. Von Bedeutung ist auch die Hemmung des Wuchses von krautigen und holzigen Pflanzen an Strassenrändern und in der Nähe von Ueberlandleitungen oder ganz allgemein in Bereichen, in denen ein starker Bewuchs unerwünscht ist.

Wichtig ist auch die Anwendung von Wachstumsregulatoren zur Hemmung des Längenwachstums bei Getreide, denn durch eine Halmverkürzung wird die Gefahr des Umknickens ("Lagerns") der Pflanzen vor der Ernte verringert oder vollkommen beseitigt. Ausserdem können Wachstumsregulatoren bei Getreide eine Halmverstärkung hervorrufen, die ebenfalls dem Lagern entgegenwirkt.

Eine Hemmung des vegetativen Wachstums erlaubt bei vielen Kulturpflanzen eine dichtere Anpflanzung der Kultur, so dass ein Mehrertrag bezogen auf die Bodenfläche erzielt werden kann.

Ein weiterer Mechanismus der Ertragssteigerung mit Wachstumshemmern beruht darauf, dass die Nährstoffe in stärkerem Masse der Blüten- und Fruchtbildung zugute kommen, während das vegetative Wachstum eingeschränkt wird.

Mit Wachstumsregulatoren lässt sich häufig auch eine Förderung des vegetativen Wachstums erzielen. Dies ist von grossem Nutzen, wenn die vegetativen Pflanzenteile geerntet werden. Eine Förderung des vegetativen Wachstums kann aber auch gleichzeitig zu einer Förderung des generativen Wachstums führen, so dass z.B. mehr oder grössere Früchte zur Ausbildung kommen.

Ertragssteigerungen können in manchen Fällen auch durch einen Eingriff in den pflanzlichen Stoffwechsel erreicht werden, ohne dass sich Aenderungen des vegetativen Wachstums bemerkbar machen. Wachstumsregulatoren können ferner eine Veränderung der Zusammensetzung der Pflanzen bewirken, so dass eine bessere Qualität der Ernteprodukte herbeigeführt wird. So ist es beispielsweise möglich, den Gehalt an Zucker in Zuckerrüben, Zuckerrohr, Ananas sowie Zitrusfrüchten zu erhöhen oder den Proteingehalt in Soja oder Getreide zu steigern.

Unter dem Einfluss von Wachstumsregulatoren kann es zur Ausbildung parthenokarper Früchte kommen. Ferner kann das Geschlecht der Blüten beeinflusst werden.

Mit Wachstumsregulatoren lässt sich auch die Produktion oder der Abfluss von sekundären Pflanzenstoffen positiv beeinflussen. Als Beispiel sei die Stimulierung des Latexflusses bei Gummibäumen genannt.

Während des Wachstums der Pflanze kann durch Einsatz von Wachstumsregulatoren auch die seitliche Verzweigung durch eine chemische Brechung der Apikaldominanz vermehrt werden. Daran besteht z.B. Interesse bei der Stecklingsvermehrung von Pflanzen. Es ist jedoch auch möglich, das Wachstum der Seitentriebe zu hemmen, z.B. um bei Tabakpflanzen nach der Dekapitierung die Ausbildung von Seitentrieben zu verhindern und damit das Blattwachstum zu fördern.

Unter dem Einfluss von Wachstumsregulatoren kann der Blattbestand von Pflanzen so gesteuert werden, dass ein Entblättern der Pflanzen zu einem gewünschten Zeitpunkt erreicht wird. Eine derartige Entlaubung ist von Interesse, um eine mechanische Beerntung, z.B. bei Wein oder Baumwolle, zu erleichtern oder um die Transpiration zu einem Zeitpunkt herabzusetzen, an dem die Pflanze verpflanzt werden soll. Durch Einsatz von Wachstumsregulatoren lässt sich der vorzeitige Fruchtfall verhindern. Es ist jedoch auch möglich, den Fruchtfall, - zum Beispiel bei Obst -, im Sinne einer chemischen Ausdünnung bis zu einem bestimmten Ausmass zu fördern. Wachstumsregulatoren können auch dazu dienen, um bei Kulturpflanzen zum Zeitpunkt der Ernte die zum Ablösen der Früchte erforderliche Kraft zu vermindern, so dass eine mechanische Beerntung der Pflanzen ermöglicht, beziehungsweise eine manuelle Beerntung erleichtert wird.

Mit Wachstumsregulatoren lässt sich ferner eine Beschleunigung oder auch eine Verzögerung der Reife des Erntegutes vor oder nach der Ernte erreichen. Dieses ist von besonderem Vorteil, weil sich dadurch eine optimale Anpassung an die Bedürfnisse des Marktes herbeiführen lässt. Weiterhin können Wachstumsregulatoren in manchen Fällen die Fruchtausfärbung verbessern. Darüberhinaus kann mit Hilfe von Wachstumsregulatoren auch eine zeitliche Konzentrierung der Reife erzielt werden. Damit werden Voraussetzungen dafür geschaffen, dass z.B. bei Tabak, Tomaten oder Kaffee, eine vollständige mechanische oder manuelle Beerntung in nur einem Arbeitsgang vorgenommen werden kann.

Durch die Anwendung von Wachstumsregulatoren kann auch die Samenoder Knospenruhe der Pflanzen, also die endogene Jahresrhythmik, beeinflusst werden, so dass die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen.

Mit Wachstumsregulatoren kann auch erreicht werden, dass der Austrieb von Knospen oder die Keimung von Samen verzögert wird, z.B. um in frostgefährdeten Gebieten eine Schädigung durch Spätfröste zu vermeiden. Andererseits gelingt es, das Wurzelwachstum zu und/oder die Ausbildung von Sprösslingen zu stimulieren, so dass das Wachstum auf eine kürzere Zeitdauer beschränkt werden kann.

Wachstumsregulatoren können auch eine Halophilie bei den Kulturpflanzen erzeugen. Damit werden die Voraussetzungen dafür geschaffen, dass eine Kultivierung von Pflanzen auf salzhaltigen Böden durchgeführt werden kann.

Mit Wachstumsregulatoren kann auch eine Frost- und Trockenresistenz bei Pflanzen induziert werden.

Unter dem Einfluss von Wachstumsregulatoren kann das Altern (die Seneszenz) von Pflanzen oder Pflanzenteilen gehemmt respektive verzögert werden. Eine solche Wirkung kann von hohem wirtschaftlichem Interesse sein, dadurch, dass bei behandelten Pflanzenteilen oder ganzen Pflanzen wie Obst, Beeren, Gemüse, Salat oder Zierpflanzen deren Lagerfähigkeit nach der Ernte verbessert oder verlängert werden kann. Ebenso

kann durch Behandlung von Kulturpflanzen über eine Verlängerung der Phase photosynthetischer Aktivität eine beachtliche Ertragssteigerung erzielt werden.

Ein weiteres wichtiges Anwendungsgebiet für Wuchshemmer ist deren Einsatz zur Hemmung eines übermässigen Wachstums bei tropischen Bodenbeckungspflanzen, den sogenannten Covet crops. In tropischen und subtropischen Monokulturen, wie z.B. in Palmplantagen, Baumwoll-, Maisfeldern usw. werden neben den eigentlichen Kulturpflanzen oftmals Bodenbedeckungspflanzen, insbesondere Leguminosenarten angepflanzt, die zur Erhaltung oder Steigerung der Bodenqualität (Verhinderung der Austrocknung, Versorgung mit Stickstoff) und zur Verhinderung von Erosion (Abtragung durch Wind und Wasser) dienen. Durch Applikation der erfindungsgemässen Wirkstoffe kann nunmehr das Wachstum dieser Cover crops kontrolliert und somit die Wuchshöhe dieser Bodenbedeckungspflanzen auf einem niedrigen Niveau gehalten werden, so dass ein gesundes Gedeihen der Kulturpflanzen und die Aufrechterhaltung einer günstigen Bodenbeschaffenheit gewährleistet ist.

Weiter hat es sich überraschenderweise gezeigt, dass die Aktivsubstanzen der Formel I bzw. entsprechende Mittel, ausser vorteilhaften wuchsregulierenden Eigenschaften zusätzlich ein für praktische Bedürfnisse sehr günstiges Mikrobizidspektrum aufweisen. Deshalb liegt ein weiteres Einsatzgebiet von Verbindungen der Formel I in der Bekämpfung von schädlichen Mikroorganismen, vor allem von phytopathogegen Pilzen. So besitzen die Verbindungen der Formel I eine für praktische Bedürfnisse sehr günstige kurative, präventive und systemische Wirkung zum Schutz von Pflanzen, insbesondere Kulturpflanzen, ohne diese nachteilig zu beeinflussen.

Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikroorganismen verschont bleiben.

Die Wirkstoffe sind gegen die den folgenden Klassen angehörenden phytopathogenen Pilze wirksam: Ascomyceten (z.B. Venturia, Podosphaera, Erysiphe, Monilinia, Uncinula); Basidiomyceten (z.B. die Gattungen Hemileia, Rhizoctonia, Puccinia); Fungi imperfecti (z.B. Bortrytis, Helminthosporium, Fusarium, Septoria, Cercospora und Alternaria). Ueberdies wirken die Verbindungen der Formel I systemisch. Sie können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden. Die erfindungsgemässen Wirkstoffe zeichnen sich durch besonders gute Pflanzenverträglichkeit aus.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen.

Darüberhinaus schliesst die vorliegende Erfindung auch die Herstellung agrochemischer Mittel ein, die gekennzeichnet ist durch das innige Vermischen der Aktivsubstanz mit einem oder mehrereb hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

Als Zielkulturen für die hierin offenbarten Indikationsgebiete gelten im Rahmen dieser Erfindung beispielsweise folgende Pflanzenarten: Getreide: (Weizen, Gerste, Roggen, Hafer, Reis, Sorhum und Verwandte); Rüben: (Zucker- und Futterrüben); Kern-, Stein- und Beerenobst: (Aepfel, Birnen, Pflaumen, Pfirsiche, Mandeln, Kirschen, Erd-, Him- und Brombeeren); Hülsenfrüchte: (Bohnen, Linsen, Erbsen, Soja); Oelkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer): oder Pflanzen wie Mais, Tabak, Nüsse, Kaffee, Zuckerrohr, Tee, Weintrauben, Hopfen, Bananen- und Naturkautschukgewächse. Pflanzen seien im Rahmen vorliegender Erfindung aber auch alle Arten von sonstigen Grünbewachsungen, seien es Zierpflanzen (Compositen), Grasflächen, Böschungen oder allgemeine niedrige Bodenbedeckungen (cover crops), die einer Erosion oder Austrocknung des Bodens entgegenwirken oder Bodenbedeckungen wie sie in Baum- und Staudenkulturen (Obstplantagen, Hopfenkulturen, Maisfeldern, Weingärten usw.) erwünscht sind.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein, Es können aber auch selektive Herbizide, Insektizide, Fungizide, Bakterizide, Nenatizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf das Blattwerk (Blattapplikation). Anzahl der Applikationen richten sich dabei nach dem Befallsdruck für den entsprechenden Erreger (Pilzsorte) oder der Art der Wachstumsbeeinflussung. Die Wirkstoffe der Formel I können aber auch

über den Erdboden durch das Wurzelwerk in die Pflanzen gelangen (systemische Wirkung), indem man den Standort der Pflanzen mit einer flüssigen Zubereitung tränkt oder die Substanzen in fester Form in den Boden einbringt, z.B. in Form von Granulat (Bodenapplikation). Die Verbindungen der Formel I können aber auch auf Samenkörner aufgebracht werden (Coating), indem man die Körner entweder mit einer flüssigen Zubereitung des Wirkstoffs tränkt oder sie mit einer festen Zubereitung beschichtet. Darüberhinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmitteln eingesetzt und werden daher z.B. zu Emulsionskonzentraten, streichfähigen Pasten, direkt versprühbaren oder verdünnbaren Lösungen, verdünnten Emulsionen, Spritzpulvern, lösliche Pulvern, Stäubemitteln, Granulaten, durch Verkapselungn in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 10 g bis 5 kg Aktivsubstanz (AS) je ha; bevorzugt 100g bis 2 kg AS/ha, insbesondere bei 200 g bis 600 g AS/ha.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctyiphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid, sowie gegegenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberfläcenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyllaur-nsalze zu erwähnen. >>äufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkalirest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierhier gehören auch die Salze der Schwefelsäureester und Sulfonsäuren von Fettalkohol-Ethylenoxyd-Addukten. Die sulfonierten Benzimidazolderivate enthalten, vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit I bis 5 Ethylenglykoleinheiten.

Als Beispiele nichtionischer Tenside seien Nonylphenolpolyethoxyethanole, Ricinusölpolyglycolether, Polypropylen-Polyethylenoxydaddukte, Tributylphenoxypolyethoxyethanol, Polyethylenglykol und Octylphenoxypolyethoxyethanol erwähnt.

Ferner kommen auch Fettsäureester von Polyoxyethylensorbitam wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammonium-bromid.

Die in der Formulierungstechnik gebräuchlichen Tenside sind u.a. in folgenden Publikationcn beschrieben:

"Mc Cutcheon's Detergents and Emulsifiers Annual" MC Publishing Corp., Ringwood New Jersey, 1980

Sisely and Wood, "Encyclopedia of Surface Active Agents", Chemical Publishing Co., Inc. New York, 1980.

Die agrochemischen Zubereitungen enthalten in der Regel 0,1 bis 99%, insbesondere 0,1 bis 95% Wirkstoff der Formel I, 99,9 bis 1%, insbesondere 99,8 bis 5% eines festen oder flüssigen Zusatzstoffes und 0 bis 25%, insbesondere 0,1 bis 25% eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung, ohne dieselbe zu beschränken. Temperaturen sind in Celsiusgraden angegeben. Prozente und Teile beziehen sich auf das Gewicht. RT bedeutet Raumtemperatur, h steht für Stunde, min für Minute, DMSO für Dimethylsulfoxid, THF für Tetrahydrofuran, DMF für Dimethylformamid.

## Herstellungsbeispiele

**Beispiel 1:** Herstellung von

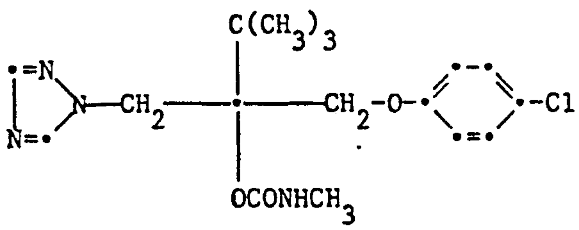

1-(4-Chlorphenoxy)-2-tert.-butyl-2-(N-Methylcarbamoyloxy)-3-[1H-1,2,4-triazol]-propan.

Man erhitzt unter Rückfluss während 15 Stunden ein Gemisch von 7,7 g (0.025 Mol) 1-(4-Chlorphenoxy)-2-tert.-butyl-2-hydroxy-3-(1H-1,2 4-triazol)-propan, 1,6 g (0.0028 Mol) Methylisocyanat und 2,8 g (0.0028 Mol) Triäthylamin in 100 ml Dioxan. Das Lösungsmittel wird dann abdestilliert, der Rückstand in Aether aufgenommen und mit Wasser ausgeschüttelt. Die Aetherphase wird über Natriumsulfat getrocknet und dann eingeengt worauf ein gelbes Oel zurückbleibt, das beim Zerreibem in Petroläther kristallisiert. Man erhält so 1,9 g (20.7% d.Theorie) Titelprodukt mit einen Schmelzpunkt von 159-162° C.

**Beispiel 2:** Herstellung von

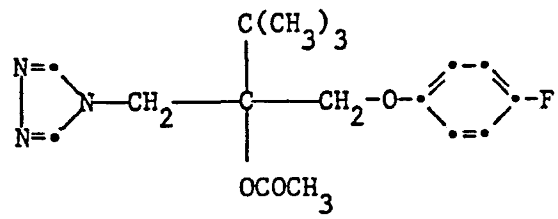

1-(4-Fluorphenoxy)-2-tert.-butyl-2-acetyloxy-3-(4H-1,2,4-triazolyl)-propan.

Man tropft eine durch Erwärmen von 10 g l-(4-Fluorphenoxy)-2-tert.-butyl-2-hydroxy-3-(4H-1,2,4-triazolyl)-propan in 50 ml Dimethylformamid erhaltene Lösung zu einer Suspension von 1,6 g Natriumhydrid (als 50%ige Oel-Dispersion) in 30 ml Tetrahydrofuran. Anschliessend wird während ca. 1/2 Stunde auf 40°C erwärmt, bis keine Wasserstoffentwicklung mehr festzustellen ist. Dann werden 3 g Acetylchlorid in das Reaktionsgemisch getropft und dieses während ca. 14 Stunden bei Rückflusstemperatur gerührt. Danach giesst man das Ganze auf ein Gemisch aus Na-bicarbonat und Eis. Die organische Phase wird mit Aethylacetat extrahiert, mit Salzsole und Wasser gewaschen und über Natriumsulfat getrocknet. Nach dem Abdampfen des Lösungsmittels verbleibt ein kristallin anfallendes Rohprodukt. Dieses wird in Aethylacetat gelöst, filtriert. Nach dem Verdampfen des Lösungsmittels verbleiben 3,6 g reines Titelprodukt vom Smp. 118-120°C.

Analyse ber. C 61,07% H 6,33% N 12,5% F 5,68%

gef. C 60,5% H 6,5% N 12,2% F 5,7%

Analog zu diesem Beispiel wurde auch l-(4-Fluorphenoxy)-2-t.-butyl-2-acetyloxy-3-(1H-1,2,4-triazolyl)-propan hergestellt, welches als zähes Oel anfällt.

**Beispiel 3:** Herstellung von

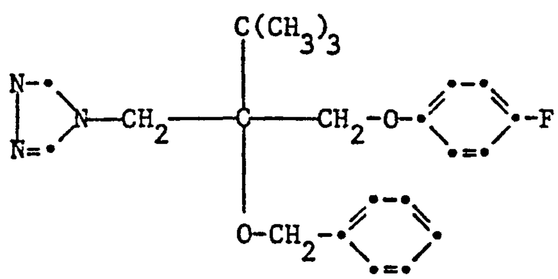

1-(4-Fluorphenoxy)-2-tert.-butyl-2-benzyloxy-3-(4H-1,2,4-triazolyl)-propan.

Man tropft eine, durch Erwärmen von 10 g 1-(4-Fluorphenoxy)-2-tert.-butyl-2-hydroxy-3-(4H-1,2,4-triazolyl)-propan in 60 ml Dimethylformamid erhaltene Lösung zu einer Suspension von 1,6 g Natriumhydrid (als 50%ige Oel-Dispersion) in 30 ml Tetrahydrofuran. Anschliessend wird während ca. 1/2 Stunde auf 40°C erwärmt bis keine Wasserstoffentwicklung mehr festzustellen ist. Dann tropft man 6,1 g Benzylbromid zum Reaktionsgemisch, rührt während ca. 14 Stunden bei 50°C und giesst das Ganze in Wasser. Die organische Phase wird mit Aethylacetat extrahiert und mehrmals mit Salzsole und Wasser gewaschen. Dann wird über Natriumsulfat getrocknet, und das Lösungsmittel abgedampft. Es verbleibt ein öliger Rückstand, der beim Stehen kristallisiert. Dieser wird über Nacht bei Raumtemperatur mit Aethylacetat gerührt. Nach Filtrieren und dem Verdampfen des Lösungsmittels verbleiben 5,5 g Titelprodukt vom Smp. 140-141°C.

Analyse ber. C 68,91% H 6,83% N 10,96% F 4,95%

gef. C 68,9% H 6,8% N 10,7% F 5,0%.

**Beispiel 4:** Herstellung von

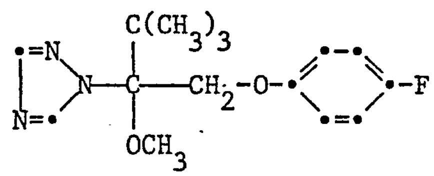

( bekannt)

1-(4-Fluorphenoxy)-2-tert.-butyl-2-methoxy-3-[1H-1,2,4-triazolyl]-propan.

Man reinigt 2,2 g Natriumhydrid durch Waschen mit Petroläther und dekantieren desselben, bevor man es in Suspension mit 45 ml Dimethylformanid gibt. Dazu tropft man bei Raumtemperatur unter Stickstoffatmosphäre und Rühren innerhalb von 15 Minutem eine Lösung von 14 g (0.05 Mol) 1-(4-Fluorphenoxy-2-tert.-butyl-2-hydroxy-3-[4H-1,2,4-triazolyl]-propan in 14 ml Tetrahydrofuran abs. und 43 ml Tetrahydrofuram abs. Die Reaktion verläuft schwach exothern. Nachdem alles zugetropft ist wird bei 40°C 1/2 Stunde weitergerührt, bis die Wasserstoffentwicklung aufgehört hat. Man kühlt dann ab und tropft innerhalb von 15 Minuten 3,45 ml (0.05 Mol) Methyljodid zum Reaktionsgemisch. Die Reaktion verläuft lebhaft, exotherm. Man lässt während 5 Stunden bei Raumtemperatur ausrühren. Dann wird das Reaktionsgemisch auf eine 1/2 gesättigte wässrige Natriumchloridlösung gegossen. Die organische Phase word 2-3 mal mit Aethylacetat extrahiert, dann gewaschen, über Natriumsulfat getrocknet, filtriert, vom Lösungsmittel befreit und im Hochvakuum destilliert. Man erhält 12,7 g (68% der Theorie) Titelprodukt als gelbes visköses Oel. Siedepunkt 245-250°C/0.04 mbar.

**Beispiel 5:** Herstellung von

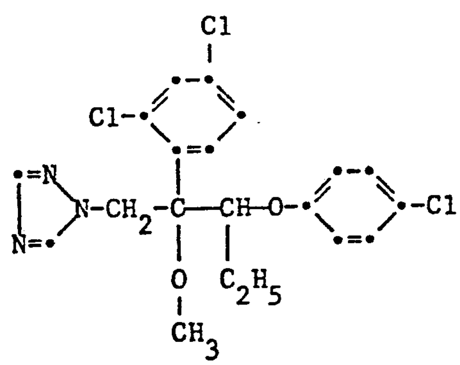

1-(1H-1,2,4-Thiazolyl)-2-methoxy-2-(2,4-dichlorphenyl)-3-(4-chlor-phenoxy)-pentan.

Zu einer vom Mineralöl befreiten (durch Waschen mit Pentan) Suspension von 1,2 g Natriumhydrid (55%ig) in 20 ml abs. Dimethylformamid gibt man tropfenweise unter Stickstoffatmosphäre eine Lösung von 9,4 g 1-(1H-1,2,4-Triazolyl)-2-hydroxy-2-(2,4-dichlorphenyl)-3-(4-chlor-phenoxy)-pentan in 30 ml Dimethylformamid. Nachdem die exotherme Reaktion und Stickstoffentwicklung abgeklungen sind, rührt man das Reaktionsgemisch noch eine Stunde bei 40°C und tropft dann 1,7 ml Methyljodid zu. Man rührt weitere 4-5 Stunden bei 40-50°C und giesst dass Reaktionsgemisch dann auf Eis. Das wässrige Gemisch wird mit Kochsalz gesättigt und dann mit Aether und Aethylacetat extrahiert. Die Extrakte werden getrocknet filtriert und eingedampft. Es verbleiben 7,5 g eines viskosen Oeles, das bei 230°C (Siedebadtemperatur) und 0,6 millibar destilliert. Die analytischen und spektrographischen Daten entsprechen den für die Titelverbindung angenommenen Struktur.

Das als Ausgangsprodukt verwendete 1-(1H-1,2,4-Triazolyl-2-hydroxy-2-(2,4-dichlorphenyl)-3-(4-chlorphenoxy)-pentan wurde wie folgt hergestellt:

a) Herstellung von 2-(2,4-Dichlorphenyl)-2-[1-(4-chlorphenoxy)-propyl]-oxiran (Zwischenprodukt)

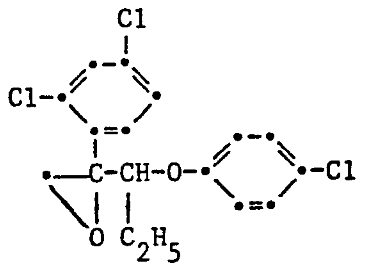

Eine Suspension von 3,0 g Natriumhydrid (55%ig in Mineralöl) wird unter Stickstoffatmosphäre mit 50 ml Petroläther gerührt, der überstehende Petroläther wird abdekantiert und der Rückstand mit 65 ml absolutem Dimethylsulfoxid versehen. Anschliessend wird dazu unter Rühren portionenweise 15,4 g Trimethyloxosulfoniumjodid getropft. Die Reaktion verläuft exotherm. Nach Beendigung der Zugabe rührt man noch 20-30 Minuten bei Raumtemperatur, bevor man eine weitere Lösung von 22 g α-(4-Chlorphenoxy)-2,4-dichlorbutyrophenon in 50 ml Dimethylsulfoxid zugetropft. Das Reaktionsgemisch wird 2 Stunden bei 55-60°C gerührt und anschliessend in 200 ml Eis/Wasser gegossen. Das wässrige Gemisch wird mit Kochsalz gesättigt und dann mit Aethylacetat extrahiert. Die organische Phase wird dreimal mit Kochsalzlösung gewaschen, getrocknet filtriert und eingedampft. Man erhält 22 g eines hellen Oeles, das nach Dünnschichtchromatographie einheitlich ist und dessen IR- und NMR-Spektrum mit den für die angegebene Oximstruktur übereinstimmen.

b) Herstellung von 2-(1H-1,2,4-Triazolyl)-2-hydroxy-2-(2,4-dichlor-phenyl)-3-[4-chlorphenoxy]-pentan (Zwischenprodukt)

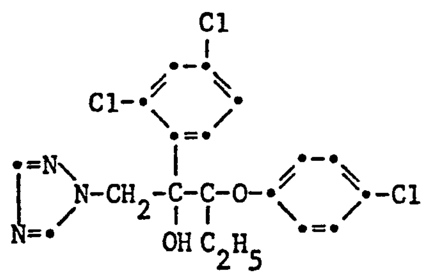

Zu einer Lösung von 2 g Kalium-tert.Butanolat und 8,8 g 1,2,4-Triazol in 70 ml absolutem Dimethylformamid tropft man eine Lösung von 2-(2,4-Dichlorphenyl)-2-[1-(4-chlorphenoxy)-propyl]-oxiran in 60 ml absolutem Dimethylformamid. Man rührt das Reaktionsgemisch während 8 Stunden bei 90-100°C, kühlt ab und gibt 350 ml 10%ige wässrige Kochsalzlösung dazu. Dann extrahiert man mit Aethylacetat, wäscht die organische Phase mit Kochsalzlösung, trocknet, filtriert und dampft sie ein. Das verbleibende Oel kristallisiert nach dem Zerreiben mit Aether/Petroläther. Man erhält 14 g weisse Kristalle vom Schmelzpunkt 133-134°C deren Analysendaten und Spektren mit der für die Titelverbindung angenommenen Struktur übereinstimmen.

In analoger Weise zu diesen Beispielen werden die nachfolgenden Verbindungen der Formel I und Ausgangsprodukte der Formel IV hergestellt:

**Tabelle 1**

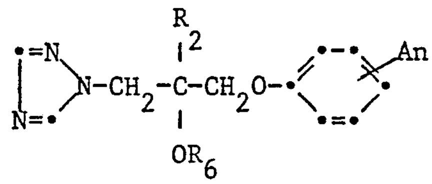

| No. | $R_2$ | $R_6$ | An | |
|---|---|---|---|---|
| 1 | $C_4H_9t$ | $CH_2CH{=}CH_2$ | Cl(4) | |
| 2 | $C_4H_9t$ | $C_4H_9n$ | Cl(4) | |
| 3 | $C_4H_9t$ | $CH_3$ | Cl(4) | Oel |
| 4 | $C_4H_9t$ | $CH_2C{\equiv}CH$ | Cl(4) | Oel |
| 5 | $C_4H_9t$ | $CH_2C_6H_5Cl(4)$ | Cl(4) | |
| 6 | $C_4H_9t$ | $CH_2C_6H_4Cl(4)$ | Cl(4) | Sdp. 220°/0.04 mbar |
| 7 | $C_3H_7i$ | $CH_2CH{=}CH_2$ | Cl(4) | |
| 8 | $C_3H_7i$ | $C_2H_5$ | Cl(4) | |
| 9 | $C_3H_7i$ | $CH_3$ | Cl(4) | $n_D^{25}$ = 1,5299 |
| 10 | $C_3H_7i$ | $CH_2C_6H_4Cl(4)$ | Cl(4) | |
| 11 | $C_3H_7i$ | $CH_2C_6H_4Cl(2)$ | Cl(4) | |
| 12 | $C_3H_7i$ | $CH_2C_6H_5$ | Cl(4) | |
| 13 | $C_4H_9t$ | $Si(CH_3)_3$ | Cl(4) | |
| 14 | $C_4H_9t$ | $CH_2C_6H_5$ | Cl(4) | |
| 15 | $C_4H_9t$ | $CH(CH_3)OC_4H_9n$ | Cl(4) | |
| 16 | $C_4H_9t$ | Tetrahydropyran-2-yl | Cl(4) | |
| 17 | $C_4H_9t$ | $CH_2C_6H_4F(4)$ | Cl(4) | |
| 18 | $C_4H_9t$ | $CH_2C_6H_4CF_3(3)$ | Cl(4) | |
| 19 | $C_3H_7i$ | $Si(CH_3)_3$ | Cl(4) | |
| 20 | $C_3H_7i$ | $CH_2C_6H_4F(4)$ | Cl(4) | |
| 21 | $C_4H_9t$ | $CH_3$ | $C_3H_7i(4)$ | Oel |
| 22 | $C_4H_9t$ | $CH_2CH{=}CH_2$ | $Cl_2(2,4)$ | Oel |
| 23 | $C_4H_9t$ | $CH_3$ | F(4) | Oel (bekannt) |
| 24 | $C_4H_9t$ | $Si(CH_3)_3$ | F(4) | |
| 25 | $C_4H_9t$ | $CH_2C_6H_5$ | F(4) | Smp. 98-100° |
| 26 | $C_4H_9t$ | $CH_2C_6H_4Cl(4)$ | F(4) | |

| No. | $R_2$ | $R_6$ | An |
|---|---|---|---|
| 27 | $C_4H_9t$ | $CH_2C_4H_6Cl(2)$ | F(4) |
| 28 | $C_3H_7i$ | $CH_3$ | $CF_3(4)$ $n_D^{25} = 1{,}4934$ |
| 29 | $C_3H_7i$ | $CH_2CH{=}CH_2$ | F(4) |
| 30 | $C_3H_7i$ | $CH_2C{\equiv}CH$ | F(4) |
| 31 | $C_3H_7i$ | $CH_2C_6H_4Cl(4)$ | F(4) |
| 32 | $C_4H_9t$ | $CH_3$ | $(CH_3)_2(2,3)$ |
| 33 | $C_4H_9t$ | $CH_2CH{=}CH_2$ | $(CH_3)_2(2,3)$ |
| 34 | $C_4H_9t$ | $CH_2C{\equiv}CH$ | $(CH_3)_2(2,3)$ |
| 35 | $C_4H_9t$ | $CH_2C_6H_4Cl(4)$ | $(CH_3)_2(2,3)$ |
| 36 | $C_4H_9t$ | $CH_3$ | CH=CH-CH=CH(~~2,3~~ 3,4) Sdp.220°/0.04 mbar |
| 37 | $C_4H_9t$ | $CH_2CH{=}CH_2$ | - |
| 38 | $C_4H_9t$ | $CH_3$ | - Sdp.200-210°/0.08 mbar |
| 39 | $C_4H_9t$ | $CH_2C_6H_5$ | CH=CH-CH=CH(3,4) Oel |
| 40 | $C_4H_9t$ | $CH_2CH{=}CH_2$ | $OCF_3(4)$ |
| 41 | $C_4H_9t$ | $CH_2C{\equiv}CH$ | $OCF_3(4)$ viskoses Oel |
| 42 | $C_4H_9t$ | $CH_3$ | $C_3H_7i$ Oel |
| 43 | $C_4H_9t$ | $CH_2C_6H_4Cl(4)$ | $OCF_3(4)$ |
| 44 | $C_4H_9t$ | $CH_3$ | $CF_3(4)$ Oel |
| 45 | $C_4H_9t$ | $CH_2CH{=}CH_2$ | $CF_3(4)$ |
| 46 | $C_4H_9t$ | $CH_2C_6H_4Cl(4)$ | $CF_3(4)$ |
| 47 | $C_4H_9t$ | $CH_3$ | $CH_3(4)$ |
| 48 | $C_4H_9t$ | $CH_2CH{=}CH_2$ | Br |
| 49 | $C_4H_9t$ | $CH_3$ | Br(4) |
| 50 | $C_4H_9t$ | $CH_2C_6H_4Cl(4)$ | Br(4) |
| 51 | $C_4H_9t$ | $CH_3$ | $OCHF_2(4)$ |
| 52 | $C_4H_9t$ | $CH_2CH{=}CH_2$ | $OCHF_2(4)$ |
| 53 | $C_4H_9t$ | $CH_3$ | $SCHF_2(4)$ |
| 54 | $C_4H_9t$ | $CH_2C_6H_4Cl(4)$ | $SCHF_2(4)$ |
| 55 | $C_4H_9t$ | $CH_3$ | Cl(3) |
| 56 | $C_4H_9t$ | $CH_2CH{=}CH_2$ | Cl(3) |
| 57 | $C_4H_9t$ | $CH_3$ | Cl(2) |
| 58 | $C_4H_9t$ | $CH_2C_6H_4Cl(4)$ | Cl(2) |

| No. | $R_2$ | $R_6$ | An |
|---|---|---|---|
| 59 | $C_4H_9t$ | $CH_3$ | $OCHF_2(3)$ |
| 60 | $C_4H_9t$ | $CH_2C_6H_4Cl(4)$ | $OCHF_2(3)$ |
| 61 | $C_4H_9t$ | $CH_3$ | $NO_2(4)$ |
| 62 | $C_4H_9t$ | $CH_3$ | $OCH_3(4)$ |
| 63 | $C_4H_9t$ | $CH_2CH{=}CH_2$ | $OCH_3(4)$ |
| 64 | $C_6H_3Cl_2(2,4)$ | $CH_3$ | - |
| 65 | $C_6H_3Cl_2(2,4)$ | $CH_2C_4H_6F(4)$ | F(4) |
| 66 | $C_6H_3Cl_2(2,4)$ | $CH_2CH{=}CH_2$ | Cl(4) |
| 67 | $C_4H_9s$ | $CH_3$ | Cl(4) |
| 68 | $C_4H_9s$ | $CH_2CH{=}CH_2$ | Cl(4) |
| 69 | $C_4H_9s$ | $CH_2C_6H_5$ | Cl(4) |
| 70 | $C_4H_9s$ | $CH_3$ | F(4) |
| 71 | $C_4H_9s$ | $C_2H_5$ | F(4) |
| 72 | $C_4H_9s$ | $CHC{\equiv}CH$ | F(4) |
| 73 | $C_4H_9i$ | $CH_3$ | - |
| 74 | $C_4H_9i$ | $CH_3$ | Cl(4) |
| 75 | $C_4H_9i$ | $C_2H_5$ | Cl(4) |
| 76 | $C_4H_9i$ | $Si(CH_3)_3$ | Cl(4) |
| 77 | $C_4H_9i$ | $CH_2C_6H_4Cl(4)$ | Cl(4) |
| 78 | $C_4H_9i$ | $CH_3$ | F(4) |
| 79 | $C_4H_9i$ | $CH_3CH{=}CH_2$ | F(4) |
| 80 | $(CH_3)_3CCH_2$ | $CH_3$ | Cl(4) |
| 81 | $(CH_3)_3CCH_2$ | $CH_2C_6H_4F(4)$ | Cl(4) |
| 82 | $(CH_3)_3CCH_2$ | $CH_2C_6H_4Cl(3)$ | Cl(4) |
| 83 | $(CH_3)_3CCH_2$ | $CH_3$ | Cl(4) |
| 84 | $C_3H_7CH(CH_3)$ | $CH_3$ | Cl(4) |
| 85 | $C_3H_7CH(CH_3)$ | $CH_2CH{=}CH_2$ | Cl(4) |
| 86 | $C_3H_7CH(CH_3)$ | $CH_3$ | F(4) |
| 87 | $C_2H_5CH(CH_3)CH_2$ | $CH_3$ | Cl(4) |
| 88 | $C_2H_5CH(CH_3)CH_2$ | $CH_3$ | F(4) |
| 89 | $(CH_3)_2CHC_2H_4$ | $CH_3$ | - |
| 90 | $(CH_3)_2CHC_2H_4$ | $CH_2C_6H_5$ | Cl(4) |

| No. | $R_2$ | $R_6$ | An | |
|---|---|---|---|---|
| 91 | $(CH_3)_2CHC_2H_4$ | $CH_3$ | F(4) | |
| 92 | $(CH_3)_2CHC_2H_4$ | $CH_2C_6H_4Cl(4)$ | F(4) | |
| 93 | $(C_2H_5)_2CH$ | $CH_3$ | Cl(4) | |
| 94 | $(C_2H_5)_2CH$ | $CH_3$ | F(4) | |
| 95 | $(C_2H_5)_2CH$ | $CH_3$ | Br(4) | |
| 96 | $C_2H_5CH(CH_3)CH(CH_3)$ | $CH_2C_6H_5$ | F(4) | |
| 97 | $C_2H_5CH(CH_3)CH(CH_3)$ | $CH_3$ | Cl(4) | |
| 98 | $C_2H_5CH(CH_3)CH(CH_3)$ | $CH_3$ | Br(4) | |
| 99 | $C_5H_{11}n$ | $CH_3$ | F(4) | |
| 100 | $C_5H_{11}n$ | $Si(CH_3)_3$ | Cl(4) | |
| 101 | $C_5H_{11}n$ | $CH_3$ | $Cl_2$(2,4) | |
| 102 | $C_6H_{13}n$ | $CH_3$ | $CF_3$(4) | $n_D^{30}$ = 1,4813 |
| 103 | $C_6H_{13}n$ | $COCH_3$ | Br(4) | $n_D^{30}$ = 1,5208 |
| 104 | $C_7H_{15}n$ | $CH_3$ | F(4) | |
| 105 | $C_7H_{15}n$ | $CH_3$ | Cl(4) | |
| 106 | $C_7H_{15}n$ | $CH_2C_6H_5$ | Cl(4) | |
| 107 | $C_8H_{17}n$ | $CH_3$ | Cl(4) | |
| 108 | $C_8H_{17}n$ | $CH_3$ | Br(4) | |
| 109 | $C_8H_{17}n$ | $CH_3$ | F(4) | |
| 110 | $C_9H_{19}n$ | $CH_3$ | Cl(4) | |
| 111 | $C_9H_{19}n$ | $CH_3$ | Cl(4) | |
| 112 | $C_9H_{19}n$ | $CH_2C_6H_5$ | Cl(4) | |
| 113 | $C_{10}H_{21}n$ | $CH_3$ | Cl(4) | |
| 114 | $C_{10}H_{21}n$ | $CH_3$ | F(4) | |
| 115 | $C_4H_9CH(C_2H_5)$ | $CH_3$ | Cl(4) | |
| 116 | $C_4H_9CH(C_2H_5)$ | $CH_3$ | F(4) | |
| 117 | Cyclohexyl | $CH_3$ | F(4) | |
| 118 | Cyclohexyl | $CH_3$ | Cl(4) | |
| 119 | 1-Methylcyclohexyl | $CH_3$ | Cl(4) | |
| 120 | 1-Methylcyclohexyl | $CH_3$ | F(4) | |
| 121 | $C_6H_3Cl_2$(2,4) | $CH_3$ | F(4) | |
| 122 | $C_3H_7i$ | $CONHCH_3$ | Cl(4) | |

| No. | $R_2$ | $R_6$ | An | |
|---|---|---|---|---|
| 123 | $C_3H_7i$ | $CON(CH_3)_2$ | F(4) | |
| 124 | $C_3H_7i$ | $CON(CH_3)_2$ | Br(4) | |
| 125 | $(CH_3)_3CCH_2$ | $CONHCH_3$ | Cl(4) | |
| 126 | $(CH_3)_3CCH_2$ | $COOC_2H_5$ | F(4) | |
| 127 | $C_2H_5CH(CH_3)$ | $COOC_2H_5$ | Cl(4) | |
| 128 | $C_2H_5CH(CH_3)$ | $CONHCH_3$ | F(4) | |
| 129 | $(CH_3)_2CH(CH_2)$ | $CONHCH_3$ | Cl(4) | |
| 130 | $(CH_3)_2CH(CH_2)$ | $CONHCH_3$ | F(4) | |
| 131 | $C_3H_7CH(CH_3)$ | $CONHCH_3$ | Cl(4) | |
| 132 | $C_3H_7CH(CH_3)$ | $CONHCH_3$ | F(4) | |
| 133 | $C_2H_5CH(CH_3)CH_2$ | $CONHCH_3$ | Cl(4) | |
| 134 | $C_2H_5CH(CH_3)CH_2$ | $COOC_2H_5$ | F(4) | |
| 135 | $(CH_3)_2CHC_2H_4$ | $CONHCH_3$ | Cl(4) | |
| 136 | $(CH_3)_2CHC_2H_4$ | $CONHCH_3$ | F(4) | |
| 137 | $(C_2H_5)_2CH$ | $COOCH_3$ | Cl(4) | |
| 138 | $(C_2H_5)_2CH$ | $CONHCH_3$ | F(4) | |
| 139 | $C_4H_9t$ | $CONHCH_3$ | Br(4) | |
| 140 | $C_4H_9t$ | $CONHC_2H_5$ | Br(4) | |
| 141 | $C_4H_9t$ | $COOC_2H_5$ | Br(4) | |
| 142 | $C_4H_9t$ | $COOCH_3$ | Br(4) | |
| 143 | $C_4H_9t$ | $CONHCH_3$ | - | |
| 144 | $C_4H_9t$ | $CONHC_2H_5$ | - | |
| 145 | $C_4H_9t$ | $COOC_2H_5$ | - | |
| 146 | $C_4H_9t$ | $CONHCH_3$ | $(CH_3)_2(2,3)$ | |
| 147 | $C_4H_9t$ | $CONHCH_3$ | $OCH_3(4)$ | |
| 148 | $C_4H_9t$ | $CONHC_2H_5$ | $OCH_3(4)$ | |
| 149 | $C_4H_9t$ | $CONHC_2H_5$ | $OCF_3(4)$ | |
| 150 | $C_4H_9t$ | $CONHC_2H_5$ | $SCF_3(4)$ | |
| 151 | $C_4H_9t$ | $CONHC_2H_5$ | $OCHF_2(4)$ | |
| 152 | $C_4H_9t$ | $CONHC_2H_5$ | $CF_3(4)$ | viskoses Oel |
| 153 | $C_4H_9t$ | $CONHC_2H_5$ | $CH_3(4)$ | |
| 154 | $C_4H_9t$ | $CONHC_2H_5$ | CN(4) | |

| No. | $R_2$ | $R_6$ | An | |
|---|---|---|---|---|
| 155 | $C_6H_3Cl_2(2,4)$ | $CONHCH_3$ | - | |
| 156 | $C_6H_3Cl_2(2,4)$ | $CONHC_4H_9n$ | 4(Cl) | |
| 157 | $C_6H_4F(4)$ | $CONHC_2H_5$ | 4(F) | |
| 158 | $C_2H_5CH(CH_3)CH(CH_3)$ | $CONHCH_3$ | 4(Cl) | |
| 159 | $C_2H_5CH(CH_3)CH(CH_3)$ | $COOCH_3$ | 4(F) | |
| 160 | $C_5H_{11}n$ | $CONHCH_3$ | Cl(4) | |
| 161 | $C_5H_{11}n$ | $COOCH_3$ | F(4) | |
| 162 | $C_5H_{11}n$ | $CONHCH_3$ | Cl(4) | |
| 163 | $C_6H_{13}n$ | $CON(CH_3)_2$ | Cl(4) | |
| 164 | $C_6H_{13}n$ | $COOCH_3$ | F(4) | |
| 165 | $C_7H_{15}n$ | $CONHCH_3$ | Cl(4) | |
| 166 | $C_7H_{15}n$ | $CONHCH_3$ | F(4) | |
| 167 | $C_8H_{17}n$ | $CONHCH_3$ | Cl(4) | |
| 168 | $C_8H_{17}n$ | $COOC_2H_5$ | F(4) | |
| 169 | $C_9H_{19}n$ | $CONHCH_3$ | - | |
| 170 | $C_9H_{19}n$ | $COOC_2H_5$ | F(4) | |
| 171 | $C_9H_{19}n$ | $CONHCH_3$ | Cl(4) | |
| 172 | $C_{10}H_{21}n$ | $CONHC_2H_5$ | Cl(4) | |
| 173 | $C_{10}H_{21}n$ | $COOC_2H_5$ | F(4) | |
| 174 | $C_4H_9nCH(C_2H_5)$ | $CONHC_2H_5$ | Cl(4) | |
| 175 | $C_4H_9nCH(C_2H_5)$ | $COOC_2H_5$ | F(4) | |
| 176 | Cyclohexyl | $COOC_2H_5$ | Cl(4) | |
| 177 | Cyclohexyl | $CONH_2H_5$ | F(4) | |
| 178 | 1-Methylcyclopropyl | $COOCH_3$ | Cl(4) | |
| 179 | 1-Methylcyclopropyl | $CONHCH_3$ | F(4) | |
| 180 | $C_4H_9t$ | $COOCH_3$ | F(4) | Smp. 54-56° |
| 181 | $C_4H_9t$ | $CONHCH_3$ | Cl(4) | Smp.159-163° Beispiel 1 |
| 182 | $C_4H_9t$ | $COOC_2H_5$ | Cl(4) | Smp. 84-86° |
| 183 | $C_4H_9t$ | $COOCH_3$ | Cl(4) | |
| 184 | $C_4H_9t$ | $CONHC_2H_5$ | Cl(4) | viskoses Oel |
| 185 | $C_4H_9t$ | $CONHC_6H_4Cl(4)$ | Cl(4) | |
| 186 | $C_3H_7i$ | $COCH_3$ | Cl(4) | |

| No. | $R_2$ | $R_6$ | An | |
|---|---|---|---|---|
| 187 | $(CH_3)_3CCH_2$ | $COC_2H_5$ | $Cl_2(2,4)$ | |
| 188 | $C_3H_7i$ | $COC_2H_5$ | $Cl_2(2,4)$ | |
| 189 | $(CH_3)_3CCH_2$ | $COCH_3$ | - | |
| 190 | $C_2H_5CHCH_3$ | $COC_3H_7n$ | F(4) | |
| 191 | $C_2H_5CH(CH_3)$ | $COCH_3$ | Cl(4) | |
| 192 | $C_2H_5CH(CH_3)$ | $COC_2H_5$ | Br(4) | |
| 193 | $C_2H_5CH(CH_3)$ | $COCH_2OCH_3$ | Br(4) | |
| 194 | $C_3H_7nCH(CH_3)$ | $COCH_3$ | Cl(4) | |
| 195 | $C_3H_7nCH(CH_3)$ | $COC_2H_5$ | $Cl_2(2,4)$ | |
| 196 | $C_2H_5CH(CH_3)CH_2$ | $COC_3H_7n$ | Cl(4) | |
| 197 | $C_2H_5CH(CH_3)CH_2$ | $COCH_3$ | - | |
| 198 | $(CH_3)_2CHC_2H_4$ | $COC_3H_7n$ | - | |
| 199 | $(CH_3)_2CHC_2H_4$ | $COCH_3$ | Cl(4) | |
| 200 | $(C_2H_5)_2CH$ | $COCH_3$ | - | |
| 201 | $(C_2H_5)_2CH$ | $COCH_2OCH_3$ | Cl(4) | |
| 202 | $C_2H_5CH(CH_3)CH(CH_3)$ | $COCH_3$ | - | |
| 203 | $C_2H_5CH(CH_3)CH(CH_3)$ | $COCH_3$ | Cl(4) | |
| 204 | $C_5H_{11}n$ | $COCH_3$ | Cl(4) | |
| 205 | $C_6H_{13}n$ | $COC_2H_5$ | Cl(4) | |
| 206 | $C_7H_{15}n$ | $COC_3H_7n$ | - | |
| 207 | $C_8H_{17}n$ | $COCH_3$ | Cl(4) | |
| 208 | $C_9H_{19}n$ | $COC_2H_5$ | Cl(4) | |
| 209 | $C_{10}H_{21}n$ | $COCH_3$ | - | |
| 210 | $C_4H_9nCH(C_2H_5)$ | $COC_2H_5$ | Cl(4) | |
| 211 | Cyclohexyl | $COCH_3$ | Cl(4) | |
| 212 | 1-Methylcyclohexyl | $COCH_3$ | Cl(4) | |
| 213 | $C_4H_9t$ | $COCH_3$ | Cl(4) | Fp. 90-92° |
| | | | Hydrochlorid | Fp. 181-183° |
| 214 | $C_4H_9t$ | $COC_2H_5$ | Cl(4) | |
| 215 | $C_4H_9t$ | $COC_3H_7n$ | Cl(4) | |
| 216 | $C_4H_9t$ | $COCH_2OCH_3$ | Cl(4) | |
| 217 | $C_4H_9t$ | $COC_6H_4Cl(4)$ | Cl(4) | |

| No. | $R_2$ | $R_6$ | An | |
|---|---|---|---|---|
| 218 | $C_4H_9t$ | $COC_6H_5$ | Cl(4) | |
| 219 | $C_4H_9t$ | $COCH_3$ | F(4) | zähes Oel |
| 220 | $C_4H_9t$ | $COC_2H_5$ | F(4) | |
| 221 | $C_4H_9t$ | $COCH_2OCH_3$ | F(4) | |
| 222 | $C_4H_9t$ | $COCH_3$ | $OCF_3$(4) | viskoses Oel |
| 223 | $C_4H_9t$ | $COC_2H_5$ | $OCF_3$(4) | |
| 224 | $C_4H_9t$ | $COCH_3$ | $(CH_3)_2$(2,3) | |
| 225 | $C_4H_9t$ | $COC_2H_5$ | $(CH_3)_2$(2,3) | |
| 226 | $C_4H_9t$ | $COCH_3$ | Br(4) | |
| 227 | $C_4H_9t$ | $COC_2H_5$ | Br(4) | |
| 228 | $C_4H_9t$ | $COCH_2OCH_3$ | Br(4) | |
| 229 | $C_4H_9t$ | $COCH_3$ | $CF_3$(4) | Smp. 74-75° |
| 230 | $C_4H_9t$ | $COC_2H_5$ | $CF_3$(4) | |
| 231 | $C_4H_9t$ | $COCH_2OCH_3$ | $CF_3$(4) | |
| 232 | $C_4H_9t$ | $COC_4H_9n$ | $CF_3$(4) | |
| 233 | $C_4H_9t$ | $COC_3H_7n$ | $CF_3$(4) | |
| 234 | $C_4H_9t$ | $COCH_3$ | Cl(2) | |
| 235 | $C_4H_9t$ | $COC_2H_5$ | Cl(2) | |
| 236 | $C_4H_9t$ | $COCH_3$ | Cl(3) | |
| 237 | $C_4H_9t$ | $COC_3H_7n$ | Cl(3) | |
| 238 | $C_4H_9t$ | $COCH_2OCH_3$ | Cl(3) | |
| 239 | $C_4H_9t$ | $COCH_3$ | $OCHF_2$(4) | |
| 240 | $C_4H_9t$ | $COC_3H_7$ | $OCHF_2$(4) | |
| 241 | $C_4H_9t$ | $COCH_2OCH_3$ | $OCH_2F_2$(4) | |
| 242 | $C_4H_9t$ | $COCH_3$ | $OCH_3$(4) | |
| 243 | $C_4H_9t$ | $COC_3H_7n$ | $OCH_3$(4) | |
| 244 | $C_4H_9t$ | $COC_4H_9t$ | Cl(4) | |
| 245 | $C_4H_9t$ | $COC_3H_7i$ | Cl(4) | |
| 246 | $C_4H_9t$ | $COC_4H_9t$ | Cl(4) | |
| 247 | $C_4H_9t$ | $COC_5H_{11}n$ | Cl(4) | |

| No. | $R_2$ | $R_6$ | An |
|---|---|---|---|
| 248 | $C_4H_9t$ | $COC_6H_{13}n$ | Cl(4) |
| 249 | $C_4H_9t$ | $COCH_2C_6H_5$ | Cl(4) |
| 250 | $C_4H_9t$ | $COCH_2C_6H_4Cl(4)$ | Cl(4) |
| 251 | $C_4H_9t$ | $COCH_2C_6H_3Cl_2(2,4)$ | Cl(4) |
| 252 | $C_6H_3Cl_2(2,4)$ | $COCH_3$ | - |
| 253 | $C_6H_3Cl_2(2,4)$ | $COCH_2OCH_3$ | F(4) |
| 254 | $C_6H_3Cl_2(2,4)$ | $COCH_2Cl$ | Cl(4) |
| 255 | $C_6H_4F(4)$ | CO Cyclopropyl | F(4) |
| 256 | $C_3H_7i$ | $SO_2CH_3$ | - |
| 257 | $C_4H_7i$ | $SO_2N(CH_3)_2$ | Cl(4) |
| 258 | $(CH_3)_3CCH_2$ | $SO_2N(CH_3)_2$ | Cl(4) |
| 259 | $(CH_3)_3CCH_2$ | $SO_2CH_3$ | Cl(4) |
| 260 | $C_2H_5CH(CH_3)$ | $SO_2CH_3$ | F(4) |
| 261 | $C_2H_5CH(CH_3$ | $SO_2N(CH_3)_2$ | Cl(4) |
| 262 | $(CH_3)_2CHCH_2$ | $SO_2N(CH_3)_2$ | - |
| 263 | $(CH_3)_2CHCH_2$ | $SO_2CH_3$ | $Cl_2(2,4)$ |
| 264 | $C_3H_7nCH(CH_3)$ | $SO_2CH_3$ | F(4) |
| 265 | $C_3H_7nCH(CH_3)$ | $SO_2CH_3$ | F(4) |
| 266 | $C_2H_5CH(CH_3)CH_2$ | $SO_2N(CH_3)_2$ | Cl(4) |
| 267 | $C_2H_5CH(CH_3)CH_2$ | $SO_2CH_3$ | F(4) |
| 268 | $(CH_3)_2CHC_2H_4$ | $SO_2N(CH_3)_2$ | Cl(4) |
| 269 | $(CH_3)_2CHC_2H_4$ | $SO_2CH_3$ | Br(4) |
| 270 | $(C_2H_5)_2CH$ | $SO_2C_2H_5$ | Cl(4) |
| 271 | $(C_2H_5)_2CH$ | $SO_2CH_3$ | $Cl_2(2,4)$ |
| 272 | $C_2H_5CH(CH_3)CH(CH_3)$ | $SO_2N(CH_3)_2$ | F(4) |
| 273 | $C_2H_5CH(CH_3)CH(CH_3)$ | $SO_2CH_3$ | Cl(4) |
| 274 | $C_5H_{11}n$ | $SO_2C_2H_5$ | F(4) |
| 275 | $C_5H_{11}n$ | $SO_2CH_3$ | Cl(4) |
| 276 | $C_6H_{13}n$ | $SO_2N(CH_3)_2$ | Cl(4) |
| 277 | $C_6H_{13}n$ | $SO_2CH_3$ | Br(4) |
| 278 | $C_7H_{15}n$ | $SO_2C_2H_5$ | F(4) |
| 279 | $C_7H_{15}n$ | $SO_2CH_3$ | Cl(4) |

| No. | $R_2$ | $R_6$ | An |
|---|---|---|---|
| 280 | $C_8H_{17}n$ | $SO_2N(CH_3)_2$ | - |
| 281 | $C_8H_{17}n$ | $SO_2N(CH_3)_2$ | Cl(4) |
| 282 | $C_9H_{19}n$ | $SO_2CH_3$ | - |
| 283 | $C_9H_{19}n$ | $SO_2CH_3$ | F(4) |
| 284 | $C_{10}H_{21}n$ | $SO_2C_2H_5$ | Cl(4) |
| 285 | $C_{10}H_{21}n$ | $SO_2N(CH_3)_2$ | Br(4) |
| 286 | $C_4H_9nCH(CH_3)$ | $SO_2N(CH_3)_2$ | F(4) |
| 287 | $C_4H_9nCH(CH_3)$ | $SO_2N(CH_3)_2$ | Cl(4) |
| 288 | Cyclohexyl | $SO_2CH_3$ | Cl(4) |
| 289 | Cyclohexyl | $SO_2N(CH_3)_2$ | Cl(4) |
| 290 | 1-Methylcyclopropyl | $SO_2CH_3$ | Cl(4) |
| 291 | $C_4H_9t$ | $SO_2CH_3$ | Cl(4) |
| 292 | $C_4H_9t$ | $SO_2C_2H_5$ | Cl(4) |
| 293 | $C_4H_9t$ | $SO_2N(CH_3)_2$ | Cl(4) |
| 294 | $C_4H_9t$ | $SO_2NHCH_3$ | Cl(4) |
| 295 | $C_4H_9t$ | $SO_2CH_3$ | F(4) |
| 296 | $C_4H_9t$ | $SO_2N(CH_3)_2$ | F(4) |
| 297 | $C_4H_9t$ | $SO_2NHCH_3$ | F(4) |
| 298 | $C_4H_9t$ | $SO_2CH_3$ | Br(4) |
| 299 | $C_4H_9t$ | $SO_2N(CH_3)_2$ | Br(4) |
| 300 | $C_4H_9$ | $SO_2NHCH_3$ | Br(4) |
| 301 | $C_4H_9t$ | $SO_2CH_3$ | $CF_3$(4) |
| 302 | $C_4H_9t$ | $SO_2N(CH_3)_2$ | $CF_3$(4) |
| 303 | $C_4H_9t$ | $SO_2NHCH_3$ | $CF_3$(4) |
| 304 | $C_4H_9t$ | $SOCH_3$ | $OCF_3$(4) |
| 305 | $C_4H_9t$ | $SO_2N(CH_3)_2$ | $OCF_3$(4) |
| 306 | $C_4H_9t$ | $SO_2NHCH_3$ | $OCF_3$(4) |
| 307 | $C_4H_9t$ | $SO_2CH_3$ | $OCHF_2$(4) |
| 308 | $C_4H_9t$ | $SO_2N(CH_3)_2$ | $OCHF_2$(4) |
| 309 | $C_4H_9t$ | $SO_2NHCH_3$ | $OCHF_2$(4) |
| 310 | $C_4H_9t$ | $SO_2CH_3$ | - |
| 311 | $C_4H_9t$ | $SON(CH_3)_2$ | - |

| No. | $R_2$ | $R_6$ | An | |
|---|---|---|---|---|
| 312 | $C_4H_9t$ | $SO_2NHCH_3$ | - | |
| 313 | $C_4H_9t$ | $SO_2CH_3$ | $OCH_3(4)$ | |
| 314 | $C_4H_9t$ | $SO_2NHCH_3$ | $OCH_3(4)$ | |
| 315 | $C_4H_9t$ | $SO_2N(CH_3)_2$ | $OCH_3(4)$ | |
| 316 | $C_4H_9t$ | $SO_2CH_3$ | Cl(2) | |
| 317 | $C_4H_9t$ | $SO_2N(CH_3)_2$ | Cl(2) | |
| 318 | $C_4H_9t$ | $SO_2NHCH_3$ | Cl(3) | |
| 319 | $C_4H_9t$ | $SO_2CH_3$ | Cl(3) | |
| 320 | $C_4H_9t$ | $SO_2N(CH_3)_2$ | Cl(3) | |
| 321 | $C_4H_9t$ | $SO_2CH_3$ | Cl(4) | |
| 322 | $C_4H_9t$ | $SO_2NHCH_3$ | Cl(4) | |
| 323 | $C_4H_9t$ | $SO_2N(CH_3)_2$ | Cl(4) | |
| 324 | $C_6H_3Cl_2(2,4)$ | $SO_2CH_3$ | F(4) | |
| 325 | $C_6H_4F(4)$ | $CH_3$ | Cl(4) | Oel |
| 326 | $C_6H_4F(4)$ | $CH_3$ | F(4) | Oel |
| 327 | $C_6H_3Cl_2(2,4)$ | $C_2H_5$ | Br(4) | Oel |
| 328 | $C_6H_4Cl_2(2,4)$ | $CH_2C{\equiv}CH$ | - | |
| 329 | $C_6H_4F(4)Cl(2)$ | $CH_3$ | Cl(4) | |
| 330 | $C_6H_4Cl_2(2,4)$ | $CH_2Cl$ | F(4) | |
| 331 | $C_4H_9t$ | $COCH_2Cl$ | Cl(4) | |
| 332 | $C_4H_9t$ | $COCF_3$ | Cl(4) | |
| 333 | $C_4H_9$ | $COC_6H_5$ | - | |
| 334 | $C_6H_3Cl_2(2,4)$ | $COCH_2Cl$ | - | |
| 335 | $C_6H_4OC_6H_5(4)$ | $CH_3$ | - | |
| 336 | $C_4H_9t$. | $COCH_3$ | $SCF_3(4)$ | viskoses Oel |
| 337 | $C_4H_9t$ | $CH_3$ | $SCF_3(4)$ | viskoses Oel |
| 338 | $C_4H_9t$ | $CON(OCH_3)_2$ | F(4) | viskoses Oel |
| 339 | β-Naphthyl | $CH_3$ | $Cl_2(3,4)$ | Oel |
| 340 | β-Naphthyl | $CH_2C_6H_4Cl(4)$ | F(4) | viskoses Oel |

**Tabelle 2**

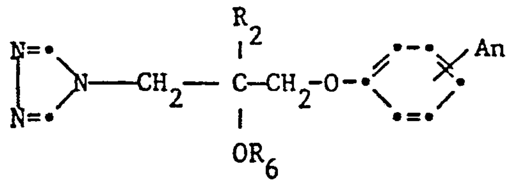

| No. | $R_2$ | $R_6$ | |
|---|---|---|---|
| 401 | $C_4H_9$t | $C_2H_5$ | Cl(4) |
| 402 | $C_3H_7$t | $CH_2CH{=}CH_2$ | Cl(4) |
| 403 | $C_3H_7$i | $CH_2C_6H_5$ | Cl(4) |
| 404 | $C_4H_7$i | $CH(CH_3)OC_4H_9$ | Cl(4) |
| 405 | $C_3H_7$i | $Si(CH_3)_3$ | Cl(4) |
| 406 | $C_4H_9$t | $CH_2C{\equiv}CH$ | Cl(4) |
| 407 | $C_4H_9$t | $CH_2C_6H_4Cl$(4) | F(4) |
| 408 | $C_4H_9$t | $CH_3$ | $(CH_3)_2$(2,3) |
| 409 | $C_4H_9$t | $CH_2C_6H_5$ | CH=CH-CH=CH(2,3) |
| 410 | $C_4H_9$t | $CH_3$ | $OCF_3$(4) |
| 411 | $C_4H_9$t | $CH_2CH{=}CH_2$ | $OCHF_3$(4) |
| 412 | $C_6H_3Cl_2$(2,4) | $CH_3$ | Br(4) |
| 413 | $C_4H_9$s | $C_2H_5$ | F(4) |
| 414 | $(C_2H_5)_2CH$ | $CH_3$ | Br(4) |
| 415 | $C_6H_{13}$n | $CH_2C_6H_4Cl$(4) | (Cl)4 |
| 416 | Cyclohexyl | $CH_3$ | Cl(4) |
| 417 | $C_3H_7$i | $CONHCH_3$ | Cl(4) |
| 418 | $C_3H_7$i | $COOC_2H_5$ | Cl(4) |
| 419 | $C_4H_9$t | $COOCH_3$ | - |
| 420 | $(C_2H_5)_2CH$ | $CONHC_2H_5$ | - |
| 421 | $C_4H_9$t | $CONHC_4H_9$t | Cl(4) |
| 422 | $C_8H_{17}$n | $CONHCH_3$ | Cl(4) |
| 423 | $C_{10}H_{21}$n | $CONHC_2H_5$ | F(4) |
| 424 | $C_3H_7$i | $COCH_3$ | Cl(4) |
| 425 | $(CH_3)_3CCH_2$ | $COC_2H_5$ | $Cl_2$(2,4) |
| 426 | $C_2H_5CH(CH_3)$ | $COC_3H_7$n | F(4) |
| 427 | $C_6H_{13}$n | $COCH_2OCH_3$ | Cl(4) |

| No. | $R_2$ | $R_6$ | An |
|---|---|---|---|
| 428 | Cyclohexyl | $COCH_3$ | Cl(4) |
| 429 | $C_4H_9t$ | $CH_2C_6H_4Cl(4)$ | Cl(4) |
| 430 | $C_4H_9t$ | $COCH_2OCH_3$ | Br(4) |
| 431 | $C_4H_9t$ | $COC_4H_9n$ | $CF_3(4)$ |
| 432 | $C_4H_9t$ | $CH_2C_6H_3Cl_2(2,4)$ | Cl(4) |
| 433 | $C_4H_9t$ | $COC_6H_{13}n$ | Cl(4) |
| 434 | $C_4H_9t$ | $SO_2CH_3$ | - |
| 435 | $C_4H_9t$ | $SO_2N(CH_3)_2$ | - |
| 436 | $C_4H_9t$ | $SO_2NHCH_3$ | - |
| 437 | $C_3H_7i$ | $SO_2CH_3$ | Cl(4) |
| 438 | $C_3H_7i$ | $SO_2N(CH_3)_2$ | Cl(4) |
| 439 | $C_3H_7i$ | $SO_2NCH_3$ | Cl(4) |
| 440 | $C_4H_9n$ | $SO_2CH_3$ | F(4) |
| 441 | $C_6H_{13}n$ | $SO_2N(CH_3)_2$ | F(4) |
| 442 | $C_8H_{17}n$ | $SO_2NHCH_3$ | F(4) |
| 443 | $C_{10}H_{21}n$ | $SO_2N(CH_3)_2$ | $OCH_3(4)$ |
| 444 | Cyclohexyl | $SO_2N(CH_3)_2$ | Cl(4) |
| 445 | $(C_2H_5)_2CH$ | $SO_2CH_3$ | Cl(4) |
| 446 | $(C_2H_5)_2CH$ | $SO_2C_2H_5$ | - |
| 447 | $(C_2H_5)_2CH$ | $SO_2N(CH_3)_2$ | $Cl_2(3,4)$ |
| 448 | $C_3H_7i$ | $SO_2NHCH_3$ | $OCF_3(4)$ |
| 449 | $C_4H_9t$ | $COCH_3$ | F(4) Smp.118-120° Beispiel 2 |
| 450 | $C_4H_9t$ | $CH_2C_6H_5$ | F(4) Smp.140-141° Beispiel 3 |
| 451 | $C_4H_9t$ | $CH_3$ | F(4) Smp. 69-71°C |

Tabelle 3

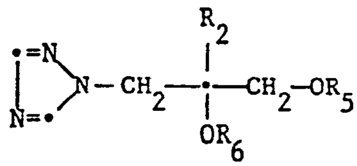

| No. | $R_2$ | $R_6$ | $R_5$ |
|---|---|---|---|
| 501 | $C_4H_9t$ | $CH_2C_6H_5Cl(4)$ | $C_4H_9n$ |
| 502 | $C_4H_9t$ | $CH_3$ | $CH_2C_6H_4Cl(4)$ |
| 503 | $C_6H_3Cl_2(2,4)$ | $CH_3$ | $C_4H_9n$ |
| 504 | $C_6H_3Cl_2(2,4)$ | $CH_2C_6H_5$ | $C_4H_9n$ |
| 505 | $C_6H_3Cl_2(2,4)$ | $CH_2C_6H_4Cl(4)$ | $C_2H_5$ |
| 506 | $C_6H_3Cl_2(2,4)$ | $CH_2C_6H_8Cl_2(2,4)$ | $CH_3$ |
| 507 | $C_6H_4C_6H_5(4)$ | $CH_3$ | $C_4H_9n$ |
| 508 | $C_6H_4C_6H_5(4)$ | $CH_2C_6H_5$ | $CH_3$ |
| 509 | $C_6H_4OC_6H_5(4)$ | $CH_3$ | $C_3H_7n$ |
| 510 | $C_4H_9t$ | $COC_6H_5$ | $C_4H_9n$ |
| 511 | $C_6H_4C_6H_5(4)$ | $COC_2H_5$ | $C_4H_9n$ |
| 512 | $C_4H_9t$ | $CH_3$ | $CH_2C_6H_5$ |
| 513 | $C_4H_9t$ | $COCH_3$ | $CH_2C_6H_5$ |
| 514 | $C_4H_9t$ | $CONHCH_3$ | $CH_2C_6H_5$ |
| 515 | $C_4H_9t$ | $COCH_3$ | $CH_2C_6H_4Cl(4)$ |
| 516 | $C_4H_9t$ | $CONHCH_3$ | $CH_2C_6H_4Cl(4)$ |
| 517 | $C_4H_9t$ | $CH_2C_6H_5$ | $CH_2C_6H_4Cl(4)$ |
| 518 | $C_4H_9t$ | $C_3H_7n$ | $CH_2C_6H_4Cl(4)$ |
| 519 | $C_4H_9t$ | $CH_3$ | $CH_2C_6H_4Cl(4)$ |
| 520 | $C_4H_9t$ | $SO_2CH_3$ | $CH_2C_6H_4Cl(4)$ |
| 521 | $C_4H_9t$ | $CH_2CN$ | $CH_2C_6H_4Cl(4)$ |
| 522 | $C_4H_9t$ | $COCH_3$ | $CH_2C_6H_4Cl(4)$ |
| 523 | $C_4H_9t$ | $CH_3$ | $CH_2C_6H_4F(4)$ |
| 524 | $C_4H_9t$ | $CONHCH_3$ | $CH_2C_6H_4F(4)$ |
| 525 | $C_4H_9t$ | $COOC_2H_5$ | $CH_2C_6H_4F(4)$ |
| 526 | $C_4H_9t$ | $C_2H_5$ | $C_4H_9n$ |

Tabelle 4

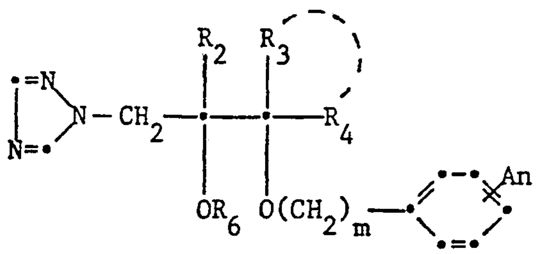

| No. | $R_2$ | $R_6$ | $R_3$ | $R_4$ | m | An |
|---|---|---|---|---|---|---|
| 601 | $C_6H_3Cl_2(2,4)$ | $CH_3$ | $C_2H_5$ | H | 0 | Smp. 86-89°C |
| 602 | $C_6H_3Cl_2(2,4)$ | $CH_3$ | $C_2H_5$ | H | 0 | Cl(4) Oel |
| 603 | $C_6H_3Cl_2(2,4)$ | $CH_3$ | $C_4H_9n$ | H | 0 | Cl(4) Oel |
| 604 | $C_6H_3Cl_2(2,4)$ | $CH_2-CH=CH_2$ | $CH_3$ | H | 0 | F(4) |
| 605 | $C_6H_3Cl_2(2,4)$ | $CH_3$ | $C_4H_9n$ | H | 0 | F(4) Sdp 230°/0.04 mbar |
| 606 | $C_6H_3Cl(2)F(4)$ | $CH_2C_6H_5$ | $CH_3$ | H | 0 | $CH_3(4)$ |
| 607 | $C_4H_9t$ | $CH_3$ | $-C_2H_4-$ | | 0 | Cl(4) |
| 608 | $C_4H_9t$ | $CH_3$ | $-C_5H_{10}-$ | | 0 | Cl(4) |
| 609 | $C_4H_9t$ | $COCH_3$ | $-C_5H_{10}-$ | | 0 | Cl(4) |
| 610 | $C_4H_9t$ | $CH_3$ | $CH_3$ | H | 0 | Cl(4) |
| 611 | $C_4H_9t$ | $C_3H_7n$ | $CH_3$ | H | 0 | Cl(4) |
| 612 | $C_4H_9t$ | $COCH_3$ | $CH_3$ | H | 0 | Cl(4) |
| 613 | $C_4H_9t$ | $CONHCH_3$ | $CH_3$ | H | 0 | Cl(4) |
| 614 | $C_4H_9t$ | $SO_2CH_3$ | $CH_3$ | H | 0 | Cl(4) |
| 615 | $C_3H_7i$ | $COCH_3$ | $CH_3$ | H | 0 | Cl(4) |
| 616 | $C_3H_7i$ | $CH_3$ | $CH_3$ | H | 0 | F(4) |
| 617 | $C_3H_7i$ | $COCH_3$ | $C_2H_5$ | H | 0 | F(4) |
| 618 | $C_4H_9t$ | $CH_2C_6H_5$ | $C_3H_7n$ | H | 0 | Cl(4) |
| 619 | $C_4H_9t$ | $CH_3$ | $C_4H_9n$ | H | 0 | Cl(4) |
| 620 | $C_4H_9t$ | $COCH_3$ | $C_4H_9n$ | H | 0 | Cl(4) |

**Tabelle 4** (Fortsetzung)

| No. | $R_2$ | $R_6$ | $R_3$ | $R_4$ | m | An |
|---|---|---|---|---|---|---|
| 621 | $C_4H_9t$ | $CH_3$ | $CH_3$ | $CH_3$ | 0 | F(4) |
| 622 | $C_4H_9t$ | $CH_3$ | $CH_3$ | $CH_3$ | 0 | Cl(4) |
| 623 | $C_4H_9t$ | $COCH_3$ | $CH_3$ | $CH_3$ | 0 | F(4) |
| 624 | $(CH_3)_2CHC_2H_4$ | $CH_3$ | $CH_3$ | H | 0 | Cl(4) |
| 625 | $(CH_3)_2CHC_2H_4$ | $CH_3$ | $CH_3$ | H | 0 | F(4) |
| 626 | $C_4H_9t$ | $CH_3$ | $CH_3$ | H | 1 | Cl(4) |
| 627 | $C_4H_9t$ | $COCH_3$ | $CH_3$ | H | 1 | Cl(4) |
| 628 | $C_4H_9t$ | $CH_3$ | $C_2H_5$ | H | 1 | Cl(4) |
| 629 | $C_4H_9t$ | $CH_3$ | $C_2H_5$ | H | 1 | F(4) |
| 630 | $C_4H_9t$ | $C_2H_5$ | $C_2H_5$ | H | 1 | F(4) |
| 631 | $C_4H_9t$ | $SO_2NHCH_3$ | $CH_3$ | H | 1 | Cl(4) |
| 632 | $C_6H_3Cl_2(2,4)$ | $CH_3$ | $C_2H_5$ | H | 0 | F(4) Sdp.250°/0.04 mbar |
| 633 | $C_6H_3Cl_2(2,4)$ | $CH_2C_6H_5$ | $CH_3$ | H | 0 | - Oel |
| 634 | $C_6H_3Cl_2(2,4)$ | $CH_2C_6H_5$ | $CH_3$ | H | 0 | Cl(4) Sdp.220°/ 0.03 mbar |
| 635 | $C_6H_3Cl_2(2,4)$ | $CH_3$ | $C_2H_5$ | H | 0 | $Cl_2$(3,5)Br(4) Smp. 47-48° |
| 636 | $C_6H_4Cl_2(2,4)$ | $CH_2C_6H_5$ | $C_4H_9n$ | H | 0 | Cl(4) Smp.86-87° |
| 637 | $C_6H_3Cl_2(2,4)$ | $CH_2C_6H_5$ | $C_4H_9n$ | H | 0 | F(4) Oel |

**Tabelle 5**

Ausgansprodukte gemäss Formel IV

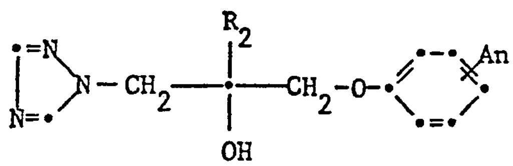

| No. | $R_2$ | An | |
|---|---|---|---|
| 701 | $C_4H_9t$ | Cl(4) | Smp. 90-92° |
| 702 | $C_4H_9t$ | Cl(2,4 | Smp. 127-128° |
| 703 | $C_4H_9t$ | F(4) | Smp. 69-70° |
| 704 | $C_4H_9t$ | $CF_3$(3) | Smp. 89-90° |
| 705 | $C_4H_9t$ | Cl(2)Br(4) | Smp. 109-110° |
| 706 | $C_4H_9t$ | - | Smp. 85-86° |
| 707 | $C_4H_9t$ | CH=CH-CH=CH(3,4) | Smp. 129-130° |
| 708 | $C_4H_9t$ | $C_6H_5$(4) | Smp. 122-124° |
| 709 | $C_4H_9t$ | $CH_2C_6H_5$(4) | Oel |
| 710 | $C_4H_9t$ | $OC_6H_5$(4) | Oel |
| 711 | $C_4H_9t$ | $OCH_2C_6H_5$(4) | Smp. 82-83° |
| 712 | $C_4H_9t$ | Cl(4) | Harz |
| 713 | $C_4H_9t$ | [$OC_6H_4CF_3$(4)](4) | Oel |
| 714 | $CH_3$ | $(CH_3)_2$(2,6) | Sdp. 178-180°/0.07 mbar |
| 715 | $CH_3$ | $C_6H_5$(4) | Smp. 112-115° |
| 716 | $CH_3$ | Cl(2)Br(4) | Smp. 130-131° |
| 717 | $CH_3$ | CH=CH-CH=CH(3,4) | Smp. 138-139° |
| 718 | $CH_3$ | $(CF_3)$(3) | Smp. 30° |
| 719 | $CH_3$ | $OCH_2C_6H_5$(4) | Smp. 122-123° |
| 720 | $CH_3$ | Cl(4) | Harz |

**Tabelle 5** (Fortsetzung)

| No | $R_2$ | An | |
|---|---|---|---|
| 721 | $CH_3$ | $[OC_6H_4CF_3(4)](4)$ | Oel |
| 722 | $CH_3$ | CH=CH-CH=CH(1,2) | Oel |
| 723 | $CH_3$ | $C_3H_7$iso(4) | Smp. 86-87° |
| 724 | $C_6H_3Cl_2(2,4)$ | Cl(4) | Smp. 150-151° |
| 725 | $C_6H_3Cl_2(2,4)$ | $Cl_2(2,4)$ | Smp. 149-150° |
| 726 | $C_6H_3Cl_2(2,4)$ | - | Smp. 120-122° |
| 727 | $C_6H_5$ | F(4) | Oel |
| 728 | $C_6H_5$ | Cl(4) | Oel |
| 729 | $C_6H_5$ | - | Smp. 108-110° |
| 730 | $C_6H_3Cl_2(2,4)$ | F(4) | Smp. 137-138° |
| 731 | $C_6H_3Cl_2(2,4)$ | $CF_3(3)$ | Smp. 113-115° |
| 732 | $C_4H_9t$ | $CH_3(2)Cl(4)$ | Smp. 126-129° |
| 733 | $C_4H_9t$ | $CH_3(4)$ | Smp. 121-122° |
| 734 | $C_4H_9t$ | $CH_3(2)$ | Smp. 92-94° |
| 735 | $C_4H_9t$ | Cl(2) | Smp. 109-110° |
| 736 | $C_6H_4Cl(4)$ | Cl(4) | Smp. 81-83° |
| 737 | $C_4H_9t$ | Cl(3) | Smp. 71-73° |
| 738 | $C_4H_9t$ | Cl(2)F(4) | Smp. 129-132° |
| 739 | $C_4H_9t$ | $Cl_2(3,4)$ | Smp. 121-124° |
| 740 | $C_4H_9t$ | $[C_6H_4Cl(4)](4)$ | Smp. 109-111° |
| 741 | $C_4H_9t$ | $OCH_3(4)$ | Smp. 63-65° |
| 742 | $C_4H_9t$ | $C(CH_3)_3(4)$ | Smp. 75-78° |
| 743 | $C_4H_9t$ | $OCF_3(4)$ | Oel |

**Tabelle 6**

Ausgangsprodukte gemäss Formel IV

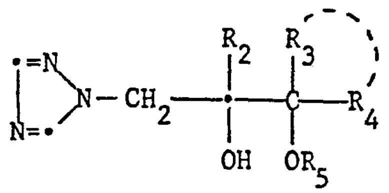

| No. | $R_2$ | $R_3$ | $R_4$ | $R_5$ | |
|---|---|---|---|---|---|
| 801 | $C_4H_9t$ | H | H | $C_4H_9n$ | Smp. 47-48° |
| 802 | $CH_3$ | $CH_3$ | H | $C_6H_4Cl(4)$ | Smp. 93-94° |
| 803 | $CH_3$ | $CH_3$ | H | $C_6H_3Cl_2(2,4)$ | Smp. 94-95° |
| 804 | $CH_3$ | $CH_3$ | H | $C_6H_3Cl_2(3,4)$ | Smp. 126-128° |
| 805 | $CH_3$ | $CH_3$ | H | $C_6H_4C_6H_5(4)$ | Smp. 134-135° |
| 806 | $C_4H_9t$ | $CH_3$ | H | $C_6H_4Cl(4)$ | Smp. 108-109° |
| 807 | $CH_3$ | $CH_3$ | H | $C_6H_4C_3H_7i(4)$ | Oel |
| 808 | $CH_3$ | $CH_3$ | H | $C_2H_4C_6H_4CH_3(2)$ | Oel |
| 809 | $C_3H_7i$ | $CH_3$ | H | $C_6H_4F(4)$ | Oel |
| 810 | $C_3H_7i$ | H | H | $C_6H_4Cl(4)$ | Smp. 108-112° |
| 811 | $C_4H_9t$ | H | H | $C_6H_4Cl(4)$ | Smp. 90-92° |
| 812 | $C_4H_9t$ | H | H | $C_6H_3Cl_2(2,4)$ | Smp. 127-128° |
| 813 | $C_4H_9t$ | H | H | $C_6H_4F(4)$ | Smp. 69-70° |
| 814 | $C_4H_9t$ | H | H | $C_6H_4CF_3(4)$ | Smp. 89-90° |
| 815 | $C_4H_9t$ | H | H | $C_6H_3Cl(2)Br(3)$ | Smp. 109-110° |
| 816 | $C_4H_9t$ | H | H | $C_6H_5$ | Smp. 85-86° |
| 817 | $C_4H_9t$ | H | H | β-Naphthyl | Smp. 129-130° |
| 818 | $C_4H_9t$ | H | H | 4-Biphenyl | Smp. 122-124° |
| 819 | $C_4H_9t$ | H | H | 4-Benzylphenyl | Oel |
| 820 | $C_4H_9t$ | H | H | 4-Phenoxyphenyl | Oel |
| 821 | $C_4H_9t$ | H | H | 4-Benzyloxy-phenyl | Smp. 82-83° |

Tabelle 6 (Fortsetzung)

| No. | $R_2$ | $R_3$ | $R_4$ | $R_5$ | |
|---|---|---|---|---|---|
| 822 | $C_4H_9t$ | H | H | $C_4H_9n$ | Smp. 47-48° |
| 823 | $C_4H_9t$ | H | H | $C_6H_4OC_6H_4CF_3(4)$ | Oel |
| 824 | $CH_3$ | H | H | $C_6H_3(CH_3)_2(2,6)$ | Sdp. 178-180°/ 0,07 mbar |
| 825 | $CH_3$ | H | H | 4-Biphenyl | Smp. 112-115° |
| 826 | $CH_3$ | H | H | $C_6H_3Cl(2)Br(4)$ | Smp. 131-132° |
| 827 | $CH_3$ | H | H | β-Naphthyl | Smp. 138-139° |
| 828 | $CH_3$ | H | H | $C_6H_4CF_3(3)$ | Smp. 30° |
| 829 | $CH_3$ | H | H | 4-Benzyloxyphenyl | Smp. 122-123° |
| 830 | $CH_3$ | H | H | $C_6H_4OC_6H_4CF_3(4)$ | Oel |
| 831 | $CH_3$ | H | H | α-Naphthyl | Oel |
| 832 | $CH_3$ | H | H | $C_6H_4C_3H_7i(4)$ | Smp. 86-87° |
| 833 | $C_3H_7i$ | $CH_3$ | H | $C_6H_5$ | Oel |
| 834 | $C_3H_7i$ | $CH_3$ | H | 4-Biphenyl | |
| 835 | $C_6H_3Cl_2(2,4)$ | H | H | $C_6H_4Cl(4)$ | Smp. 150-151° |
| 836 | $C_6H_3Cl_2(2,4)$ | H | H | $C_6H_3Cl_2(2,4)$ | Smp. 149-150° |
| 837 | $C_6H_3Cl_2(2,4)$ | H | H | $C_6H_5$ | Smp. 120-122° |
| 838 | $C_6H_5$ | H | H | $C_6H_4F(4)$ | Oel |
| 839 | $C_6H_5$ | H | H | $C_6H_4Cl(4)$ | Oel |
| 840 | $C_6H_5$ | H | H | $C_6H_5$ | Smp. 108-110° |
| 841 | $C_6H_3Cl_2(2,4)$ | H | H | $C_6H_4F(4)$ | Smp. 137-138° |
| 842 | $C_6H_3Cl_2(2,4)$ | H | H | $C_6H_4CF_3(4)$ | Smp. 113-115° |
| 843 | $C_6H_3Cl_2(2,4)$ | H | H | $C_4H_9n$ | Oel |
| 844 | 4-Biphenyl | H | H | $C_6H_5$ | Smp. 154-155° |
| 845 | 4-Biphenyl | H | H | $C_4H_9n$ | Oel |

**Tabelle 6** (Fortsetzung)1

| No. | $R_2$ | $R_3$ | $R_4$ | $R_5$ | |
|---|---|---|---|---|---|
| 846 | $C_9H_9t$ | H | H | $C_6H_4Cl(3)$ | Smp. 81-83° |
| 847 | $C_4H_9t$ | H | H | $C_6H_4Cl(2)$ | Smp. 116-117° |
| 848 | $C_4H_9t$ | H | H | $C_6H_4F(2)$ | Oel |
| 849 | $C_4H_9t$ | H | H | $C_6H_3(CH_3)_2(2,3)$ | Smp. 132-133° |
| 850 | $C_4H_9t$ | H | H | $C_6H_3(OCH_3)_2(2,6)$ | Smp. 109-110° |
| 851 | $C_4H_9t$ | H | H | $C_6H_2(CH_3)_2(3,5)Cl(4)$ | Smp. 115-116° |
| 852 | $C_4H_9t$ | H | H | $C_6H_4C_3H_7i(4)$ | Oel |
| 853 | $C_4H_9t$ | H | H | $C_6H_4CH_2-CH=CH_2(2)$ | Smp. 85-87° |
| 854 | $C_4H_9t$ | H | H | $C_6H_3Cl_2(2,3)$ | Smp. 136-137° |
| 855 | $C_4H_9t$ | H | H | $C_6H_4F(3)$ | Oel |
| 856 | $C_4H_9t$ | H | H | α-Naphthyl | Smp. 136-138° |
| 857 | $C_4H_9t$ | H | H | $C_6H_3CH_3(2)Cl(4)$ | Smp. 110-111° |
| 858 | $C_4H_9t$ | H | H | $C_6H_4Br(4)$ | Smp. 93-95° |
| 859 | $C_4H_9t$ | H | H | $C_6H_4OCHF_2(4)$ | Oel |
| 860 | $C_4H_9t$ | H | H | $C_6H_4OCF_3(4)$ | |
| 861 | $C_6H_4F(4)$ | H | H | $C_6H_4F(4)$ | Oel |
| 862 | $C_6H_4F(4)$ | H | H | $C_6H_4Cl(4)$ | Oel |
| 863 | $C_6H_4Br$ | H | H | $C_6H_4H_{11}(4)$ (Cyclo-hexyl) | Smp. 152-154° |
| 864 | $C_6H_4Br$ | H | H | 4-Biphenyl | Smp. 113-114° |
| 865 | $C_6H_3Cl_2(2,4)$ | H | H | $C_6H_4CH_3(4)$ | Smp. 124-126° |
| 866 | $C_6H_3Cl_2(2,4)$ | H | H | $C_6H_4F(2)$ | Smp. 141-142° |
| 867 | $C_6H_3Cl_2(2,4)$ | H | H | $C_6H_4Cl(2)Br(4)$ | Smp. 153-154° |
| 868 | $C_6H_3Cl_2(2,4)$ | H | H | $C_6H_2Cl_3(2,4,5)$ | Smp. 124-125° |
| 869 | $C_6H_3Cl_2(2,4)$ | H | H | $C_6H_3Br(2)Cl(4)$ | Smp. 143-144° |
| 870 | $C_6H_3Cl_2(2,4)$ | H | H | $C_6H_2(CH_3)_2(2,6)$ | |

**Tabelle 6** (Fortsetzung)

| No. | $R_2$ | $R_3$ | $R_4$ | $R_5$ | |
|---|---|---|---|---|---|
| 871 | $C_6H_4Br(4)$ | H | H | $C_6H_4CH_2$-CH=$CH_2$(2) | Oel |
| 872 | $C_6H_3Cl_2(2,4)$ | H | H | $C_6H_4CN(4)$ | Smp. 197-199° |
| 873 | $C_6H_3Cl_2(2,4)$ | H | H | $C_6H_4Cl(2)$ | Smp. 155-156° |
| 874 | $C_6H_3Cl(2)F(4)$ | H | H | $C_6H_4Cl(4)$ | Smp. 112-114° |
| 875 | $C_6H_3Cl_2(2,4)$ | $C_2H_5$ | H | $C_6H_4Cl(4)$ | Smp. 132-133° |
| 876 | $C_6H_3Cl_2(2,4)$ | $C_2H_5$ | H | $C_6H_4F(4)$ | Smp. 139-141° |
| 877 | $C_6H_3Cl_2(2,4)$ | $C_2H_5$ | H | $C_6H_3(CH_3)_2(2,3)$ | Smp. 121-122° |
| 878 | $C_6H_3Cl_2(2,4)$ | $C_3H_7$n | H | $C_6H_4Cl(4)$ | Smp. 124-125° |
| 879 | $C_6H_3Cl_2(2,4)$ | $C_2H_5$ | H | $C_6H_5$ | Smp. 135-136° |
| 880 | $C_6H_3Cl_2(2,4)$ | $C_2H_5$ | H | $C_6H_4Br(4)$ | Smp. 121-124° |
| 881 | $C_6H_3(CH_3)_2(2,4)$ | H | H | $C_6H_4Cl(4)$ | Smp. 98-99° |
| 882 | $C_6H_3Cl_2(2,4)$ | $C_4H_9$n | H | $C_6H_4F(4)$ | Smp. 174-175° |
| 883 | $C_6H_3Cl_2(2,4)$ | $C_2H_5$ | H | $C_6H_4Cl(4)$ | Smp. 150-151° |
| 884 | $C_6H_3Cl_2(2,4)$ | $CH_3$ | H | $C_6H_5$ | Smp. 150-152° |
| 885 | $C_6H_3Cl_2(2,4)$ | $CH_3$ | H | $C_6H_4Cl(4)$ | Smp. 130-132° |
| 886 | $C_6H_3(CH_3)(2,4)$ | H | H | $C_6H_3Cl_2(3,4)$ | Oel |
| 887 | Naphthyl-2 | H | H | $C_6H_3Cl_2(2,4)$ | Smp. 169-171° |
| 888 | Naphthyl-2 | H | H | $C_6H_3Cl_2(3,4)$ | Smp. 124-126° |
| 889 | $C_6H_4Cl(2)$ | H | H | $C_6H_4Br(4)$ | Smp. 50° |
| 890 | $C_4H_9$t | H | H | $C_6H_4(CH_3)(4)$ | Smp. 106-108° |
| 891 | $C_6H_3Cl_2(2,4)$ | $C_2H_5$ | H | $C_6H_4Br(4)$ | Smp. 121-124° |
| 892 | $C_6H_3Cl_2(2,4)$ | $C_2H_5$ | H | $C_6H_5$ | Smp. 155-156° |
| 893 | $C_6H_3Cl_2(2,4)$ | H | H | $C_6H_5$ | Oel |

**Tabelle 6** (Fortsetzung)

| No. | $R_2$ | $R_3$ | $R_4$ | $R_5$ | |
|---|---|---|---|---|---|
| 894 | $C_6H_3Cl_2(2,4)$ | $C_2H_5$ | H | $C_6H_4F(4)$ | Oel |
| 895 | $C_6H_3Cl_2(2,4)$ | $C_3H_7n$ | H | $C_6H_4F(4)$ | Smp. 143-145° |
| 896 | $C_6H_3Cl_2(2,4)$ | $C_2H_5$ | H | $C_6H_4(CH_3)(4)$ | Smp. 160-163° |
| 897 | $C_6H_4Cl(4)$ | Cyclo-hexyl | H | $C_6H_4F(4)$ | Smp. 50° |
| 898 | $C_6H_3Cl_2(2,4)$ | $C_3H_7n$ | H | $C_6H_3Cl(2)CF_3(4)$ | Smp. 184-185° |
| 899 | $C_6H_3Cl_2(2,4)$ | $C_2H_5$ | H | $C_6H_2(CH_3)3,5\,Br(4)$ | Smp. 155-158 |
| 900 | $C_6H_4Cl(4)$ | $C_3H_7n$ | H | $C_6H_4F(4)$ | Oel |
| 901 | $C_6H_3Cl_2(2,4)$ | $C_2H_5$ | H | $C_6H_4(CH_3)(4)$ | Smp. 98-100° |
| 902 | $C_6H_3Cl_2(2,4)$ | $CH_3$ | H | $C_6H_4F(4)$ | Smp. 121-124° |
| 903 | $C_6H_4Cl(4)$ | Cyclo-hexyl-methyl | H | $C_6H_4Cl(4)$ | Oel |
| 904 | $C_6H_4Cl(4)$ | Cyclo-hexyl | H | $C_6H_4Cl(4)$ | Smp. 160-162° |

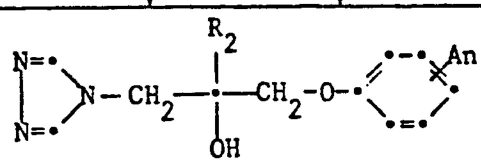

| No. | $R_2$ | An | |
|---|---|---|---|
| 905 | $C_4H_9t$ | Cl(4) | Smp. 226-228° |
| 906 | $C_4H_9t$ | F(4) | Smp. 192-193° |
| 907 | $C_4H_9t$ | $CF_3(3)$ | Smp. 217° |
| 908 | $C_4H_9t$ | - | Smp. 209-210° |
| 909 | $C_4H_9t$ | CH=CH-CH=CH | Smp. 172-173° |
| 910 | $C_4H_9t$ | $CH_2C_6H_5(4)$ | Smp. 196-197° |
| 911 | $C_4H_9t$ | $OCH_2C_6H_5(4)$ | Smp. 173-174° |
| 912 | $C_4H_9t$ | $OC_6H_5$ | Smp. 184-187° |

**Beispiel 7:**

**Formulierungsbeispiele für flüssige Wirkstoffe der Formel I (% = Gewichtsprozent)**

**Emulsions-Konzentrate** a) b) c)
Wirkstoff aus den Tabellen 1 bis 4 25% 40% 50%
Ca-Dodecylbenzolsulfonat 5% 8% 6%
Ricinusöl-polyethylenglykolether 5% -
(36 Mol Ethylenoxid)
Tributylphenoyl-polyethylenglykol- - 12% 4%
ether (30 Mol Ethylenoxid)
Cyclohexanon - 15% 20%
Xylolgemisch 65% 25% 20%
Aus solchen Konzentraten können durch Verdünnen mit Wasser-Emulsionen jeder gewünschten Konzentration hergestellt werden.
**Lösungen** a) b) c) d)
Wirkstoff aus den Tabellen 1 bis 4 80% 10% 5% 95%
Ethylenglykol-monomethyl-ether 20% - -
Polyethylenglykol M G 400 - 70% -
N-Methyl-2-pyrrolidon - 20% -
Epoxydiertes Kokosnussöl - - 1% 5%
Benzin (Siedegrenzen 160-190°C) - - 94%
(MG = Molekulargewicht)
Die Lösungen sind zur Anwendung in Form kleinster Tropfen geeignet.
**Granulate** a) b)
Wirkstoff aus den Tabellen 1 bis 4 5% 10%
Kaolin 94%- > >ochdisperse Kieselsäure 1%
Attapulgit - 90%
Der Wirkstoff wird in Methylenchlorid gelöst, auf den Träger aufgesprüht und das Lösungsmittel anschliessend im Vakuum abgedampft.
**Stäubemittel** a) b)
Wirkstoff aus den Tabellen 1 bis 4 2% 5% > >ochdisperse Kieselsäure 1% 5%
Talkum 97%
Kaolin - 90%
Durch inniges Vermischen auf Trägerstoffe mit dem Wirkstoff erhältman gebrauchsfertige Stäubemittel.

**Formulierungsbeispiele für feste Wirkstoffe der Formel 1 (% = Gewichtsprozent)**

**Spritzpulver** a) b) c)
Wirkstoff aus den Tabellen 1 bis 4 25% 50% 75%
Na-Ligninsulfonat 5% 5%
Na-Laurylsulfat 3% - 5%
Na-Diisobutylnaphthalinsulfonat - 6% 10%
Octylphenolpolyethylenglykolether - 2%
(7-8 Mol Ethylenoxid) > >ochdisperse Kieselsäure 5% 10% 10%
Kaolin 62% 27%
Der Wirkstoff wird mit den Zusatzstoffen gut vermischt und in einergeeigneten Mühle gut vermahlen. Man erhält Spritzpulver die sich mit Wasser zu Suspensionen jeder gewünschten Konzentration verdünnen lassen.

**Emulsions-Konzentrate**

Wirkstoff aus den Tabellen 1 bis 4 10%
Octylphenolpolyethylenglykolether 3%
(4-5 Mol Ethylenoxid)
Ca-Dodecylbenzolsulfonat 3%
Ricinusölpolyglykolether (35 Mol Ethylen- 4%
oxid)
Cyclohexanon 30%
Xylolgemisch 50%
Aus diesem Konzentrat können durch Verdünnen mit Wasser Emulsionen jeder gewünschten Konzentration hergestellt werden.

**Stäubemittel** a) b)
Wirkstoff aus den Tabellen 1 bis 4 5% 8%
Talkum 95%
Kaolin - 92%
Man erhält anwendungsfertige Stäubemittel, indem der Wirkstoff mitden Träger vermischt und auf einer geeigneten Mühle vermahlen wird.

**Extruder Granulate**

Wirkstoff aus den Tabellen 1 bis 4 10%
Na-Ligninsulfonat 2%
Carboxymethylcellulose 1%
Kaolin 87%
Der Wirkstoff wird mit den Zusatzstoffen vermischt, vermahlen und mit Wasser angefeuchtet. Dieses Gemisch wird extrudiert und anschliessend im Luftstrom getrocknet.

**Umhüllungs-Granulate**

Wirkstoff aus den Tabellen 1 bis 4 3%
Polyethylenglykol (M G 200) 3%
Kaolin 94%
(MG = Molekulargewicht)
Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

**Suspensions-Konzentrate**

Wirkstoff aus den Tabellen 1 bis 4 40%
Ethylenglykol 10%
Nonylphenolpolyethylenglykolether 6%
(15 Mol Ethylenoxid)
N-Lingninsulfonat 10%
Carboxymethylcellulose 1%
37%ige wässrige Formaldehyd-Lösung 0,2%
Silikonöl in Form einer 75%igen 0,8%
wässrigen Emulsion
Wasser 32%
Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

**Biologische Beispiele:**

**Beispiel 8: Wirkung gegen Puccinia graminis auf Weizen**

**a) Residual-protektive Wirkung**

Weizenpflanzen wurden 6 Tage nach der Aussaat mit einer aus Spritzpulver des Wirkstoffes hergestellten Spritzbrühe (0,002% Aktivsubstanz) besprüht. Nach 24 Stunden wurden die behandelten Pflanzen mit einer Uredosporensuspension des Pilzes infiziert. Nach einer Inkubation während 48 Stunden bei 95-100% relativer Luftfeuchtigkeit und ca 20°C wurden die infizierten Pflanzen in einem Gewächshaus bei ca 22°C aufgestellt Die Beurteilung der Rotpustelnentwicklung erfolgte 12 Tage nach der Infektion.

**b) Systemische Wirkung**

Zu Weizenpflanzen wurde 5 Tage nach der Aussaat eine aus Spritzpulver des Wirkstoffes hergestellte Spritzbrühe gegossen (0,006% Aktivsubstanz bezogen auf das Bodenvolumen). Nach 48 Stunden wurden die behandelten Pflanzen mit einer Ureidosporensuspension des Pilzes infiziert. Nach einer Inkubation während 48 Stunden bei 95-100% relativer Luftfeuchtigkeit und ca. 20°C wurden die infizierten Pflanzen in einem Gewächshaus bei ca. 22°C aufgestellt. Die Beurteilung der Rostpustelnentwicklung erfolgte 12 Tage nach der Infektion.

Verbindungen aus den Tabellen 1 bis 3 zeigten gegen Puccinia-Pilze eine gute Wirkung. Unbehandelte aber infizierte Kontrollpflanzen zeigten einen Puccinia-Befall von 100%. Unter anderem hemmten die Verbindungen der Formel I den Puccinia-Befall auf 0 bis 5%.

Die Verbindungen Nr. 3 und 36 hemmten in Test (a) sogar bei einer Verdünnung auf 0,0002% den Befall ncoh vollständig (0%).

**Beispiel 9: Wirkung gegen Cercospora arachidicola auf Erdnusspflanzen**

**Residual-protektive Wirkung**

10-15 cm hohe Erdnusspflanzen wurden mit einer aus Spritzpulver der Wirksubstanz hergestellten Spritzbrühe (0,02% Aktivsubstanz) besprüht und 48 Stunden später mit einer Konidiensuspension des Pilzes infiziert. Die infizierten Pflanzen wurden während 72 Stunden bei ca. 21°C und hoher Luftfeuchtigkeit inkubiert und anschliessend bis zum Auftraten der typischen Blattflecken in einem Gewächshaus aufgestellt. Die Beurteilung der fungiziden Wirkung erfolgte 12 Tage nach der Infektion basierend auf Anzahl und Grösse der auftretenden Flecken.

Im Vergleich zu unbehandelten, aber infizierten Kontrollpflanzen (Anzahl und Grösse der Flecken = 100%), zeigten Erdnusspflanzen, die mit Wirkstoffen aus den Tabellen 1 bis 3 behandelt wurden, einen stark reduzierten Cercospora-Befall. So verhinderten die Verbindungen der Formel I in obigen Versuchen das Auftreten von Flecken fast vollständig (0-10%). Besonders wirksam warendie Substanzen 3 und 36, die bei dieser Aufwandmenge der Cercosporabefall völlig (0%) unterdrückten.

**Beispiel 10: Wirkung gegen Erysiphae graminis auf Gerste**

**a) Residual-protektive Wirkung**

Ca. 8 cm hohe Gerstenpflanzen wurden mit einer aus Spritzpulver des Wirkstoffes hergestellten Spritzbrühe (0,002% Aktivsubstanz) besprüht. Nach 3-4 Stunden wurden die behandelten Pflanzen mit Konidien des Pilzes bestäubt. Die infizierten Gerstenpfkanzen wurden in einem Gewächshaus bei ca. 22°C aufgestellt und der Pilzbefall nach 10 Tagen beurteilt.

**b) Systemische Wirkung**

Zu ca. 8 cm hohen Gerstenpflanzen wurde eine aus Spritzpulver des Wirkstoffes hergestellte Spritzbrühe gegossen (0,002% Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit Konidien des Pilzes bestäubt. Die infizierten Gerstenpflanzen wurden in einem Gewächshaus bei ca. 22°C aufgestellt und der Pilzbefall nach 10 Tagen beurteilt.

Verbindungen der Formel I zeigten eine gute Wirkung gegen Erysiphe-Pilze. Unbehandelte, aber infizierte Kontrollpflanzen zeigten einen Erysiphe-Befall von 100%. Unter anderen Verbindungen aus den Tabellen 1 bis 3 hemmten die Verbindungen der Formel 1 z.B. Nr. 3 und Nr. 36 den Pilzbefall bei Gerste auf 0 bis 5%.

**Beispiel 11: Residual-protektive Wirkung gegen Venturia inaequalis auf Apfeltrieben**

Apfelstecklinge mit 10-20 cm langen Frischtrieben wurden mit einer aus Spritzpulver des Wirkstoffes hergestellten Spritzbrühe (0,06% Aktivsubstanz) besprüht. Nach 24 Stunden wurden die behandelten Pflanzen mit einer Konidiensuspension des Pilzes infiziert. Die Pflanzen wurden dann während 5 Tagen bei 90-100% relativer Luftfeuchtigkeit inkubiert und während 10 weiteren Tagen in einem Gewächshaus bei 20-24°C aufgestellt. Der Schorfbefall wurde 15 Tage nach der Infektion beurteilt. Verbindungen der Formel I und andere

hemmten den Krankheitsbefall auf weniger als 10%. Unbehandelte aber infizierte Triebe zeigten einen 100%igen Venturia-Befall.

**Beispiel 12: Wirkung gegen Botrytis cinerea auf Bohnen Residual protektive Wirkung**

Ca. 10 cm hohe Bohnen-Pflanzen wurden mit einer aus Spritzpulver des Wirkstoffes hergestellten Spritzbrühe (0,002% Aktivsubtanz) besprüht. Nach 48 Stunden wurden die behandelten Pflanzen mit einer Konidiensuspension des Pilzes infiziert. Nach einer Inkubation der infizierten Pflanzen während 3 Tagen bei 95.100% relativer Luftfeuchtigkeit und 21°C erfolgte die Beurteilung des Pilzbefalls. Die Verbindungen aus den Tabellen 1 bis 3 hemmten in vielen Fällen die Pilzinfektion sehr stark. Bei einer Konzentration von 0,02% erwiesen sich die Verbindungender Formel I wirksam (Krankheitsbefall 0 bis 5%). Der Botrytis-Befall unbehandelter aber infizierter Bohnenpflanzen betrug 100%.

**Beispiel 13: Wuchshemmung bei Getreide**

In Kunststofftöpfen mit sterilisierter Erde wurden die Getreidearten Hordeum vulgare (Sommergerste) und Secale (Sommerroggen) im Gewächshaus angesät und nach Bedarf bewässert. Die Sprösslinge wurden ca. 21 Tage nach der Aussaat mit der wässrigen Spritzbrühe eines Wirkstoffes der Formel I besprüht. Die Wirkstoffmenge betrug umgerechnet 0,5 bzw. 2,5 kg Aktivsubstanz pro Hektar. 10 und 21 Tage nach Applikation wurde das Wachstum des Getreides beurteilt. Hierbei konnte festgestellt werden, dass Getreidepflanzen, die mit Wirkstoffen der Formel I behandelt worden waren, im Vergleich zu unbehandelten Kontrollpflanzen eine starke Wuchsreduktion aufweisen. Als besonders wirksam erwiesen sich die Verbindungen vom Beispiel 4 und die Verbindung 3. Es wurden Zuwachsraten von weniger als 10% festgestellt.

**Beispiel 14: Wuchshemmung bei Gräsern**

In Kunststoffschalen mit Erde-Torf-Sand-Gemisch (6:3:1) wurden die Gräser Lolium perenne, Poa pratensis, Festuca orina und Cynodon dactylon im Gewächshaus angesät und nach Bedarf bewässert. Die aufgelaufenen Gräser wurden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Formel I besprüht. Die Wirkstoffmenge betrug umgerechnet 0,5 bzw. 2,5 kg Aktivsubstanz pro Hgktar. 10 und 21 Tage nach Applikation wurde das Wachstum der Gräser beurteilt, dabei zeigte es sich, dass die erfindungsgemässen Wirkstoffe aus den Tabellen 1 bis 3 eine merkliche Wuchshemmung bewirkten. Besonders deutliche Wuchshemmung wurde mit den Verbindungcn aus den Tabellen 1 und 3 bewirkt, so reduzierten die Verbindungen der Formel I Weiterwachsen fast vollständig (Zuwachsrate 0 bis 10%).

**Beispiel 15. Ertragssteigerung an Sojabohnen**

In Kunststoffbehältern mit einem Erde-Torf-Sandgemisch im Verhältnis 6:3:1 wurden Sojabohnen der Sorte "Hark" angesät und in eine Klimakammer gegeben. Durch optimale Temperaturwahl, Beleuchtung, Düngerzugabe und Bewässerung entwickelten sich die Pflanzen nach ca. 5 Wochen in dem 5-6 Trifola-Blattstadium. Zu diesem Zeitpunkt wurden die Pflanzen mit der wässrigen grühe eines Wirkstoffes der Formel I bis zur guten Benetzung besprüht. Die Wirkstoffkonzentration betrug bis zu 500 ppm Aktivsubstanz. Die Auswertung erfolgte ca. 5 Wochen nach der Applikation des Wirkstoffs. Im Vergleich zu unbehandelten Kontrollpflanzen bewirkten die erfindungsgemässen Wirkstoffe der Formel I eine merkliche Erhöhung der Anzahl und des Gewichts der geernteten Schoten. Als besonders wirksam erwiesen sich die Verbindungen aus den Tabellen 1 und 3.

**Beispiel 16: Wuchshemmung bei Bodenbedecker-Pflanzen (Cover-Crops)**

In Kunststoffschalen mit Erde-Torf-Sand-Gemisch (1:1:1) werden Testpflanzen der Sorte Centrosema plumieri und Centrosema pubescens aus Stecklingen angezogen. Nach dem Anwurzeln werden die Pflänzchen in 9-cm-Töpfe umgetopft und nach Bedarf gewässert. Die weitere Anzucht der Pflanzen findet im Gewächshaus bei einer Tagestemperatur von 27°C und einer Nachttemperatur von 21°C, bei einer mittleren Lichtdauer von 14 h (6000 Lux) und einer Luftfeuchtigkeit von 70% statt. Die Testpflanzen werden auf eine Höhe

von ca. 15 cm zurückgeschnitten und 7 Tage nach dem Zurückschneiden mit einer Spritzbrühe des Wirkstoffes (umgerechnet 0,3 bzw. 3 kg Aktivsubstanz je Hektar) besprüht. 4 Wochen nach Applikation wird das Wachstum der behandelten Pflanzen im Vergleich zu gestutzten, aber unbehandelten Kontrollpflanzen verglichen. Hierbei kann festgestellt werden, dass Verbindungen aus den Tabellen 1 bis 3 eine deutliche Wuchshemmung der Bodenbedecker-Pflanzen auslösen. Die Verbindung Nr. 3 bewirkte eine starke Wuchshemmung und reduzierte die Zuwachsrate auf weniger als 10%.

**Beispiel 17: Seneszenzhemmung bei Getreidepflanzen**

Im Gewächshaus wird Sonmerweizen der Sorte "Svenno" in Töpfen mit Komposterde angesät und ohne spezielle klimatische Anforderungen gezogen. Ca. 10 Tage nach dem Auflaufen werden 10 bis 12 cm lange Primärblätter abgeschnitten und einzeln in Reagenzgläser mit 10 ml einer Wirkstoffsuspension (1,25 bis 10 ppm Aktivsubstanz) gegeben. Die Reagenzgläser werden in einem klimatisierten Raum bei 23°C, 70% relativer Luftfeuchtigkeit aufgestellt und täglich durchschnittlich 14 Stunden (10 000 Lux) bestrahlt. Die Beurteilung der Seneszenzhemmung erfolgt 7 Tage nach Einstellen der Blätter durch Vergleich des Vergilbungsgrades im Verhältnis zu noch frischen, grünen Blättern. Bei diesem Versuch kann beobachtet werden, dass Verbindungen aus den Tabellen 1 bis 3 eine deutliche Inhibierung der Seneszenz der Testpflanzen hervorrufen. Die Verbindungen der Formel I hemmten das Vergilben der Blätter im Versuchszeitraum um mehr als 80%.

**Patentansprüche:**

1. Verbindungem der Formel I

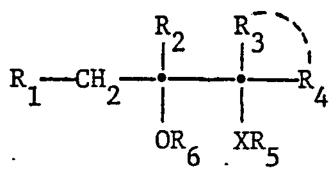

(I)

worin

$R_1$ einen über Stickstoff gebundenen Triazolrest,

$R_2$ $C_1$-$C_{10}$ Alkyl, $C_3$-$C_{10}$ Cycloalkyl, einen unsubstituiertenoder im Kern durch Halogen, Cyan, $C_1$-$C_3$ Alkyl, $C_1$-$C_3$ Haloalkyl, $C_1$-$C_3$ Alkoxy, $C_1$-$C_3$ Haloalkoxy, $C_1$-$C_3$ Alkylthio, $C_1$-$C_3$ Haloalkylthio, Nitro und/oder Rhodano substituierten Phenyl-, Naphthyl-, Biphemyl-, Phenoxyphenyl- oder Benzylrest,

$R_3$ und $R_4$ unabhängig voneinander je Wasserstoff, $C_1$-$C_4$ Alkyl oder zusammen eine 2-10 gliedrige Alkylenbrücke,

$R_5$ einen unsubstituierten oder ein- oder mehrfach substituierten Rest ausgewählt aus der Reihe $C_1$-$C_8$ Alkyl, $C_3$-$C_8$ Cycloalkyl, $C_3$-$C_6$ Alkenyl, $C_3$-$C_6$ Alkinyl, Phenyl, Naphthyl, Biphenyl, Benzylphenyl, Benzyloxyphenyl, Phenoxyphenyl und Benzyl, wobei die Substituenten aus der Reihe Halogen, Cyano, $C_1$-$C_3$ Alkyl, $C_1$-$C_5$ Alkoxy, $C_1$-$C_5$ Haloalkyl, $C_1$-$C_3$ Alkylthio, $C_1$-$C_3$ Haloalkyl, $C_1$-$C_3$ Haloalkylthio, Nitro und/oder Rhodano ausgewählt sind,

$R_6$ $C_1$-$C_{10}$ Alkyl, unsubstituiert oder substituiert durch Halogen, Cyan, $C_1$-$C_4$ Alkoxy, $C_1$-$C_4$ Alkylcarbonyl, $C_1$-$C_4$ Alkoxycarbonyl, $C_1$-$C_4$ Alkylthio, $C_1$-$C_4$ Alkylthiocarbonyl; $C_3$-$C_4$ Alkenyl, $C_3$-$C_4$Alkinyl, $C_3$-$C_8$ Cycloalkyl; einen im Kern durch Halogen, Cyan, $C_1$-$C_3$ Alkyl, $C_1$-$C_3$ Haloalkyl, $C_1$-$C_3$ Alkoxy, $C_1$-$C_3$ Haloalkoxy, $C_1$-$C_3$ Alkylthio, $C_1$-$C_3$ Haloalkylthio Nitro und/oder Rhodano substituierten, Benzylrest oder einen Rest $-Si(C_1$-$C_4$ $Alkyl)_3$, $-CONR_7R_8$, $-COR_9$, $-SO_2NR_7R_8$ oder $-SO_2R_{10}$, worin

$R_7$ und $R_8$ je einzeln Wasserstoff oder $C_1$-$C_6$ Alkyl oder zusammen mit den Stickstoffatom, an das sie gebunden sind, einen gesättigten, 5-6 gliedrigen Heterocyclus, der auch Sauerstoff, Schwefel, die Amino oder eine $C_1$-$C_4$ Alkylaminogruppe enthalten kann,

$R_9$ $C_1$-$C_{10}$ Alkyl unsubstituiert oder substituiert durch Halogen, Cyan, $C_1$-$C_4$ Alkoxy, $C_1$-$C_4$ Alkylcarbonyl, $C_1$-$C_4$ Alkoxycarbonyl, Phenyl oder $C_3$-$C_8$ Cycloalkyl oder einen unsubstituierten oder durch Halogen, Cyan, $C_1$-$C_3$ Alkyl, $C_1$-$C_3$ Haloalkyl, $C_1$-$C_3$ Alkoxy, $C_1$-$C_3$ Haloalkoxy, $C_1$-$C_3$ Alkylthio, $C_1$-$C_3$ Haloalkylthio, Nitro und/oder Rhodano substituierten Phenylrest,

$R_{10}$ $C_1$-$C_4$ Alkyl oder Phenyl, unsubstituiert oder substituiert durch Halogen, Cyan, $C_1$-$C_3$ Alkyl, $C_1$-$C_3$ Haloalkyl, $C_1$-$C_3$ Alkoxy, $C_1$-$C_3$ Haloalkoxy, $C_1$-$C_3$ Alkylthio, $C_1$-$C_3$ Haloalkylthio, Nitro und/oder Rhodano und

X Sauerstoff oder Schwefel bedeutet; unter Einschluss der Säureadditionssalze, quaternären Azoliumsalze und Metallkomplexe, mit Ausnahme von 1-[1,2,4-Triazol]-2-methoxy-2-tert.butyl-3-(4-fluorphenoxy)-propan.

2. Verbindungen gemäss Anspruch 1 der Formel Ib

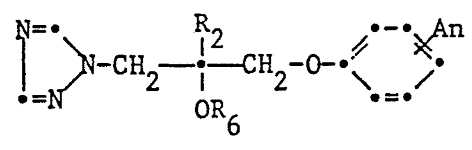

(Ib)

worin $R_2$ und $R_6$ die im Anspruch 1 gegebene Bedeutung haben, während

A Halogen, Cyan, $C_1$-$C_3$ Alkyl, $C_1$-$C_3$ Haloalkyl, $C_1$-$C_3$ Alkoxy, $C_1$-$C_3$ Haloalkoxy, $C_1$-$C_3$ Alkylthio, $C_1$-$C_3$ Haloalkylthio, Nitro oder Rhodano und

n Null oder eine Zahl von 1 bis 3 bedeuten, mit Ausnahme von 1-[1H-1,2,4-Triazol]-2-methoxy-2-tert.butyl-3-(4-fluorphenoxy)-propan.

3. Verbindungen gemäss Anspruch 1 der Formel Ij

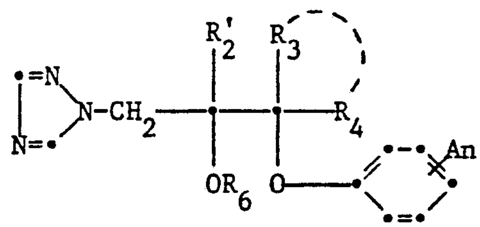

(Ij)

worin $R_3$ $R_4$ und $R_6$ die im Anspruch 1 gegebene Bedeutung haben, während

A Chlor, Cyan, $C_1$-$C_3$ Alkyl, $C_1$-$C_3$ Alkoxy, $C_1$-$C_3$ Haloalkoxy, $C_1$-$C_3$ Alkylthio, $C_1$-$C_3$ Haloalkylthio, Nitro und/oder Rhodano,

n Null oder eine Zahl von 1 bis 3, und

$R'_2$ einen $C_1$-$C_{10}$ Alkyl oder $C_1$-$C_{10}$ Cycloalkylrest bedeutet.

4. 1-(4-Chlorphenoxy)-2-tert.butyl-2-methoxy-3-[1H-1,2,4-triazolyl]-propan gemäss Anspruch 1.

5. 1-(β-Naphthyl)-2-tert.butyl-2-methoxy-3-[1H-1,2,4-triazolyl]-propan gemäss Anspruch 1.

6. Verfahren zur Herstellung der Verbindungen gemäss Anspruch 1, dadurch gekennzeichnet, dass man ein 2-Hydroxy-3- di- oder triazolylpropan-derivat der Formel IV

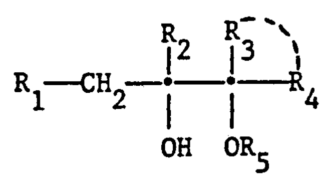

(IV)

worin $R_1$, $R_2$, $R_3$, $R_4$ und $R_5$ die im Anspruch 1 gegebene Bedeutungen haben, mit einem reaktionsfähigen Rest der Formel V

$Y$-$R_6$ (V)

worin $R_6$ die im Anspruch 1 gegebene Bedeutung hat, und Y ein Halogenatom oder einen organischen oder anorganischen Säurerest bedeutet, in einen inerten organischen Lösungs- oder Verdünnungsmittel gegebenenfalls in Gegenwart einer Base als Säurebindenden Mittel umsetzt.

7. Mittel zur Bekämpfung oder Verhütung eines Befalls durch Mikroorganismen und/oder zur Regulierung des Pflanzenwachstums, dadurch gekennzeichnet, dass es als aktive Komponente eine wirksame Menge einer Verbindung der Formel 1 gemäss Anspruch 1 enthält nebst inerten Trägerund Zusatzstoffen.

8. Verfahren zur Bekämpfung oder Verhütung eines Befalls von Kulturpflanzen durch phytopathogene Mikroorganismen und/oder zur Regulierung des Pflanzenwachstums, dadurch gekennzeichnet, dass man eine wirksame Menge einer Verbindung der Formel I, Anspruch 1, auf die Pflanze oder deren Standort appliziert.

9. Verwendung der Verbindungen der Formel I gemäss Anspruch 1 zur Bekämpfung und/oder zur präventiven Verhütung eines Befalls von Mikroorganismen und/oder zur Regulierung des Pflanzenwachstums.

10. Verwendung der Verbindungen der Formel I gemäss Anspruch 1 zur Wuchshemmung in Sinne einer Erhöhung der Knickfestigkeit und Halmverkürzung bei Getreidesorten.

11. Verwendung der Verbindungen der Formel I gemäss Anspruch 1 zur Wuchshemmung bei Bodenbedeckerpflanzen.

## Claims

1. A compound of the formula I

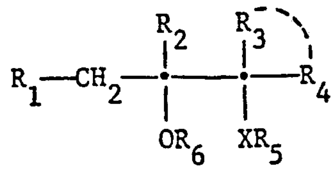

(I)

wherein

$R_1$ is a triazole radical which is bound through a nitrogen atom,

$R_2$ is $C_1$-$C_{10}$alkyl, $C_3$-$C_{10}$cycloalkyl, a phenyl, naphthyl, biphenyl, phenoxyphenyl or benzyl radical, each unsubstituted or substituted in the nucleus by a member selected from the group consisting of halogen, cyano, $C_1$-$C_3$alkyl, $C_1$-$C_3$haloalkyl, $C_1$-$C_3$alkoxy, $C_1$-$C_3$-haloalkoxy, $C_1$-$C_3$alkylthio, $C_1$-$C_3$haloalkylthio, nitro and/or thiocyanato,

$R_3$ and $R_4$ are each independently hydrogen or $C_1$-$C_4$ alkyl or together from a 2- to 10-membered alkylene bridge,

$R_5$ is an unsubstituted or mono- or polysubstituted radical selected from the group consisting of $C_1$-$C_8$alkyl, $C_3$-$C_8$cycloalkyl, $C_3$-$C_6$alkenyl, $C_3$-$C_6$alkynyl, phenyl, naphthyl, biphenyl, benzylphenyl, benzyloxyphenyl, phenoxyphenyl and benzyl, said substituents being selected from the group consisting of halogen, cyano, $C_1$-$C_3$alkyl, $C_1$-$C_5$alkoxy, $C_1$-$C_5$haloalkoxy, $C_1$-$C_3$alkylthio, $C_1$-$C_3$haloalkyl, $C_1$-$C_3$haloalkylthio, mitro and/or thiocyanato,

$R_6$ is $C_1$-$C_{10}$alkyl, unsubstituted or substituted by a member selected from the group consisting of halogen, cyano, $C_1$-$C_4$alkoxy, $C_1$-$C_4$alkylcarbonyl, $C_1$-$C_4$alkoxycarbonyl, $C_1$-$C_4$alkylthio or

$C_1$-$C_4$alkylthiocarbonyl; or is $C_3$-$C_4$alkenyl, $C_3$-$C_4$alkynyl or $C_3$-$C_8$cycloalkyl; a benzyl radical which is substituted in the nucleus by a member selected from the group consisting of halogen, cyano, $C_1$-$C_3$alkyl $C_1$-$C_3$haloalkyl $C_1$-$C_3$alkoxy, $C_1$-$C_3$halo-alkoxy, $C_1$-$C_3$alkylthio, $C_1$-$C_3$haloalkylthio nitro and/or thiocyanato, or is a -$Si(C_1$-$C_4alkyl)_3$, -$CONR_7R_8$, -$COR_9$, -$SO_2NR_7R_8$ or -$SO_2R_{10}$ radical, wherein

$R_7$ and $R_8$ are each independently hydrogen or $C_1$-$C_6$alkyl or together with the nitrogen atom to which they are attached are a saturated 5- to 6-membered heterocycle which may also contain oxygen, sulfur the amino group or a $C_1$-$C_4$alkylamino group,

$R_9$ is $C_1$-$C_{10}$alkyl, unsubstituted or substituted by a member selected from the group consisting of halogen, cyano, $C_1$-$C_4$alkoxy, $C_1$-$C_4$alkylcarbonyl, $C_1$-$C_4$alkoxycarbonyl, phenyl or $C_3$-$C_8$cycloalkyl, or is phenyl or phenyl which is substituted by a member selected from the group consisting of halogen, cyano, $C_1$-$C_3$alkyl, $C_1$-$C_3$-haloalkyl, $C_1$-$C_3$alkoxy, $C_1$-$C_3$-haloalkoxy, $C_1$-$C_3$alkylthio, $C_1$-$C_3$haloalkylthio, nitro and/or thiocyanato,

$R_{10}$ is $C_1$-$C_4$alkyl or phenyl, unsubstituted or substituted by halogen, cyano, $C_1$-$C_3$alkyl, $C_1$-$C_3$haloalkyl, $C_1$-$C_3$alkoxy, $C_1$-$C_3$ haloalkoxy, $C_1$-$C_3$alkylthio, $C_1$-$C_3$haloalkylthio nitro and/or thiocyanato and

X is oxygen or sulfur, or an acid addition salt, quaternary azolium salt or metal complex thereof, with the exception of 1-(1H-1,2,4-triazole)-2-methoxy-2-tert-butyl-3-(4-fluorophenoxy) propane.

2. A compound according to claim 1 of the formula Ib

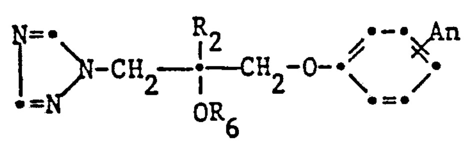

(Ib)

wherein $R_2$ and $R_6$ are as defined in claim I, and A is halogen, cyano, $C_1$-$C_3$alkyl, $C_1$-$C_3$haloalkyl, $C_1$-$C_3$alkoxy, $C_1$-$C_3$haloalkoxy, $C_1$-$C_3$alkyl-thio, $C_1$-$C_3$haloalkylthio, nitro or thiocyanato, and n is 0 or a value from 1 to 3 with

the exception of 1-[1H-1,2,4-triazole]-2-methoxy-2-tert-butyl-3-(4-fluorophenoxy)propane.

3. A compound according to claim 1 of the formula Ij

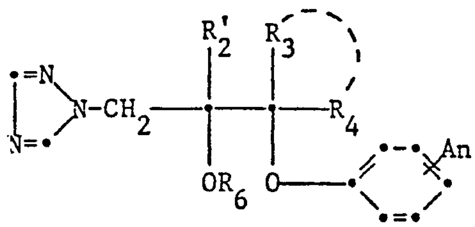

(Ij)

wherein $R_3$, $R_4$ and $R_6$ are as defined in claim 1, and A is chlorine, cyano, $C_1$-$C_3$alkyl, $C_1$-$C_3$haloalkyl, $C_1$-$C_3$alkoxy, $C_1$-$C_3$haloalkoxy, $C_1$-$C_3$alkylthio, $C_1$-$C_3$haloalkylthio, nitro and/or thiocyanato,n is 0 or a value from 1 to 3, and $R'_2$ is a $C_1$-$C_{10}$alkyl or $C_3$-$C_1$ cycloalkyl radical.

4. 1-(4-Chlorophenoxy)-2-tert-butyl-2-methoxy-3-[1H-1,2,4-triazolyl]-propane according to claim 1.

5. 1-(β-Naphthyl)-2-tert-butyl-2-methoxy-3-[1H-1,2,4-triazolyl]-propane according to claim 1.

6. A process for the preparation of a compound according to claim 1, wich process comprises reacting a 2-hydroxy-3-di- or -triazolylpropane derivative of the formula IV

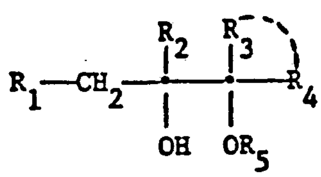

(IV)

wherein $R_1$, $R_2$, $R_3$, $R_4$ and $R_5$ are as defined in claim 1, with a reactive radical of the formula V

Y - $R_6$ (V)

wherein $R_6$ is as defined in claim 1 and Y is a halogen atom or an organic or inorganic acid radical, in an inert organic solvent or diluent and optionally in the presence of a base as acid acceptor.

7. A composition for controllimg microorganisms or preventing attack by such microorganisms, and/or for regulating plant growth, which composition contains, as active ingredient, en effective amount of a compound of formula I according to claim 1, together with inert carriers and adjuvants.

8. A method of controlling phytopathogenic microorganisms or of protecting cultivated plants from attack by such microorganisms, and/or of regulating plant growth, which comprises applying to said plants or to the locus thereof an effective anount of a compound of the fornula 1 according to claim 1.

9. Use of a compound of formula I according to claim 1 for controlling microorganisms and/or for preventing attack by such microorganisms, and/or for regulating plant growth.

10. Use of a compound of formula I according to claim 1 for inhibiting growth in order to increase resistance to lodging and to shorten the stems in crops of cereals.

11. Use of a compound of formula I according to claim 1 for inhibiting the growth of cover crops.

**Revendications**

1. Composés de formule I

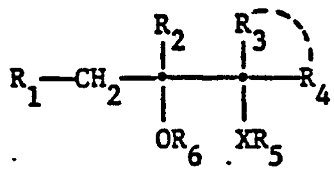 (I)

où

$R_1$ représente un radical di- ou triazole lié par l'intermédiaire d'un azote,

$R_2$ représente un alcoyle en $C_1$ à $C_{10}$, un cycloalcoyle en $C_3$ à $C_{10}$, un radical phényle, naphtyle, biphényle, phénoxyphényle ou benzyle non substitué ou substitué dans son noyau par halogène, cyano, alcoyle en $C_1$ à $C_3$, haloalcoyle en $C_1$ à $C_3$, alcoxy en $C_1$ à $C_3$, haloalcoxy en $C_1$ à $C_3$, alcoylthio en $C_1$ à $C_3$, haloalcoylthio en $C_1$ à $C_3$, nitro et/ou rhodano,

$R_3$ et $R_4$ représentent chacun indépendamment l'un de l'autre un hydrogène, un alcoyle en $C_1$ à $C_4$ ou ensemble un pont alcoylène de 2 à 10 chaînons,

$R_5$ représente un radical non substitué ou mono- ou polysubstitué, choisi dans la série alcoyle en $C_1$ à $C_8$, cycloalcoyle en $C_3$ à $C_8$, alcényle en $C_3$ à $C_6$, alcynyle en $C_3$ à $C_6$, phényle, naphtyle, biphényle, benzylphényle, benzyloxyphényle, phénoxyphényle et benzyle, où les substituants sont choisis dans la série halogène cyano, alcoyle en $C_1$ à $C_3$, alcoxy en $C_1$ à $C_5$, haloalcoxy en $C_1$ à $C_5$, alcoylthio en $C_1$ à $C_3$, haloalcoyle en $C_1$ à $C_3$, haloalcoylthio en $C_1$ à $C_3$, nitro et/ou rhodano,

$R_6$ représente un alcoyle en $C_1$ à $C_{10}$, non substitué ou substitué par halogène, cyano, alcoxy en $C_1$ à $C_4$, alcoylcarbonyle en $C_1$ à $C_4$, alcoxycarbonyle en $C_1$ à $C_4$, alcoylthio en $C_1$ à $C_4$ ou alcoylthiocarbonyle en $C_1$ à $C_4$; alcényle en $C_3$ à $C_4$, alcynyle en $C_3$ à $C_4$, cycloalcoyle en $C_3$ à $C_8$;

un radical benzyle substitué dans son noyau par halogène, cyano, alcoyle en $C_1$ à $C_3$, haloalcoyle en $C_1$ à $C_3$, alcoxy en $C_1$ à $C_3$, haloalcoxy en $C_1$ à $C_3$, alcoylthio en $C_1$ à $C_3$, haloalcoylthio en $C_1$ à $C_3$, nitro et/ou rhodano ou un radical $-Si(C_1-C_4\ alcoyl)_3$, $-CONR_7R_8$, $-COR_9$, $-SO_2NR_7R_8$ ou $-SO_2R_{10}$,

où

$R_7$ et $R_8$ représentent chacun isolément un hydrogène ou un alcoyle en $C_1$ à $C_6$ ou ensemble avec l'atome d'azote auquel ils sont liés un hétérocycle saturé de 5 à 6 chaînons, qui peut également contenir un oxygène, un soufre, le groupe imino ou un groupe alcoyle en $C_1$ à $C_4$-imino,

$R_9$ représente un acoyle en $C_1$ à $C_{10}$ non substitué ou substitué par halogène, cyano, alcoxy en $C_1$ à $C_4$, alcoylcarbonyle en $C_1$ à $C_4$, alcoxycarbonyle en $C_1$ à $C_4$, phényle ou cycloalcoyle en $C_3$ à $C_8$, ou un radical phényle non substitué ou substitué par halogène, cyano, alcoyle en $C_1$ à $C_3$, haloalcoyle en $C_1$ à $C_3$, alcoxy en $C_1$ à $C_3$ haloalcoxy en $C_1$ à $C_3$, alcoylthio en $C_1$ à $C_3$, haloalcoylthio en $C_1$ à $C_3$, nitro et/ou rhodano,

$R_{10}$ représente un alcoyle en $C_1$ à $C_4$ ou un phényle, non substitué ou substitué par halogène, cyano, alcoyle en $C_1$ à $C_3$, alcoxy en $C_1$ à $C_3$, alcoylthio en $C_1$ à $C_3$, haloalcoylthio en $C_1$ à $C_3$, nitro et/ou rhodano et

X représente un oxygène ou un soufre; y compris leurs sels d'addition d'acides, sels d'azolium quaternaires et complexes métalliques à l'exception du 1-(1H-1,2,4-triazol)-2-méthoxy-2-tert.-butyl-3-(4-fluoro-phénoxy)-propane.

2. Composés selon la rev. 1 de formule Ib

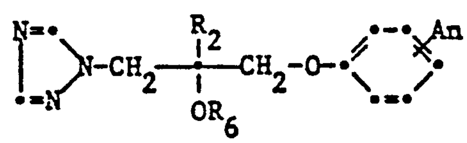 (Ib)

où $R_2$ et $R_6$ ont la signification donnée dans la rev. 1, tandis que A représente un halogène, cyano alcoyle en $C_1$ à $C_3$, haloalcoyle en $C_1$ à $C_3$, alcoxy en $C_1$ à $C_3$, haloalcoxy en $C_1$ à $C_3$, alcoylthio en $C_1$ à $C_3$, haloalcoylthio en $C_1$ à $C_3$, nitro ou rhodano et n vaut zéro ou un nombre allant de 1 à 3 à l'exception du 1-(1H-1,2,4-triazol)-2-méthoxy-2-tert.-butyl-3-(4-fluorophénoxy)-propane.

3. Composés selon la rev. 1 de formule Ij

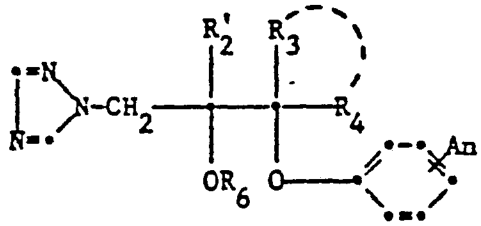

(Ij)

où $R_3$, $R_4$ et $R_6$ ont la signification donnée dans la rev. 1, tandis que

A représente un chlore, cyano, alcoyle en $C_1$ à $C_3$, haloalcoyle en $C_1$ à $C_3$, alcoxy en $C_1$ à $C_3$, haloalcoxy en $C_1$ à $C_3$, alcoylthio en $C_1$ à $C_3$, haloalcoylthio en $C_1$ à $C_3$, nitro et/ou rhodano,

n vaut zéro ou un nombre allant de 1 à 3, et $R_2'$ représente un alcoyle en $C_1$ à $C_{10}$ ou un radical cycloalcoyle en $C_3$ à $C_{10}$.

4. 1-(4-chlorophénoxy)-2-tert.-butyl-2-méthoxy-3-(1H-1 2,4-triazolyl)-propane selon la rev. 1.

5. 1-(β-naphtyl)-2-tert.butyl-2-méthoxy-3-(1H-1,2,4-triazolyl)-propane selon la rev. 1.

6. Procédé de préparation des composés selon la rev. 1, caractérisé en ce qu'on fait réagir un dérivé de 2-hydroxy-3- di- ou triazolylpropane de formule IV

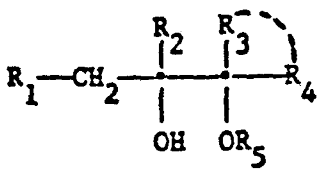

(IV)

où $R_1$, $R_2$, $R_3$, $R_4$ et $R_5$ ont les significations données dans la rev. 1, avec un radical réactif de formule V

Y-$R_6$ (V)

où $R_6$ a la signification donnée dans la rev. 1, et Y représente un atome d'halogène ou un radical acide organique ou inorganique, dans un solvant ou diluant organique inerte éventuellement en présence d'une base comme un agent liant d'acide.

7. Agent pour combattre ou prévenir une attaque par les microorganismes et/ou pour régler la croissance des plantes, caractérisé en ce qu'il contient comme composant actif une quantité efficace d'un composé de formule I selon la rev. 1, outre des supports et additifs inertes.

8. Procédé pour combattre ou prévenir une attaque des plantes cultivées par des microorganismes phytopathogènes, caractérisé en ce qu'on applique une quantité efficace d'un composé de formule I de la rev. 1 aux plantes ou à l'endroit où elles se trouvent.

9. Application des composés de formule I selon la rev. 1 pour combattre et/ou prévenir une attaque par des microorganismes et/ou pour régler la croissance des plantes.

10. Application des composés de formule I selon la rev. 1 à l'inhibition de la croissance au sens d'un accroissement de la résistance au pliage et d'un raccourcissement des tiges dans diverses variétés de céréales.

11. Application des composés de formule I selon la rev. 1 à l'inhibition de la croissance dans les plantes protectrices du sol.